# EUROPÄISCHE PATENTSCHRIFT

(11) **EP 3 174 588 B1**
(45) Veröffentlichungstag und Bekanntmachung des Hinweises auf die Patenterteilung: **11.09.2019**
(21) Anmeldenummer: 15753325.8
(22) Anmeldetag: 31.07.2015
(51) Int. Cl.: A61M 21/00

(54) **AUTOMATISCHES ERZEUGEN VON VISUELLEN STIMULI**
AUTOMATIC GENERATION OF VISUAL STIMULI
GÉNÉRATION AUTOMATIQUE DE STIMULI VISUELS

(30) Priorität: 01.08.2014 DE 102014215211
(43) Veröffentlichungstag der Anmeldung: 07.06.2017
(73) Patentinhaber: ART+COM AG, 10787 Berlin (DE); Graft Gesellschaft von Architekten mbH, 10557 Berlin (DE)
(72) Erfinder: LÜTZ, Alawi, 10117 Berlin (DE); SPIES, Claudia, 10779 Berlin (DE); HE, Jing, 10243 Berlin (DE); ÄNGESLEVÄ, Jussi, 10243 Berlin (DE); WILLEMEIT, Thomas, 10115 Berlin (DE)
(74) Vertreter: Maikowski & Ninnemann Patentanwälte Partnerschaft mbB
(86) Internationale Anmeldenummer: PCT/EP2015/067730
(87) Internationale Veröffentlichungsnummer: WO 2016/016454

(56) Entgegenhaltungen:
- EP-A1- 0 938 866
- EP-A1- 1 656 880
- WO-A1-2012/104758
- WO-A1-2012/176098
- DE-A1- 10 233 960
- DE-A1- 10 254 051
- NL-C- 2 009 753
- US-A- 5 465 729
- US-A- 5 662 117
- US-A1- 2008 242 954
- US-A1- 2009 326 616
- US-A1- 2014 316 191

## Beschreibung

### Hintergrund

Die vorliegende Erfindung betrifft eine Steuereinrichtung gemäß dem Oberbegriff des Patentanspruchs 1, eine Wiedergabeeinrichtung gemäß dem Oberbegriff des Patentanspruchs 14, sowie ein Computerprogramm zum Betreiben einer Wiedergabeeinrichtung gemäß dem Patentanspruch 15.

Insbesondere betrifft die vorliegende Erfindung ein automatisches Erzeugen von visuellen Stimuli mittels einer Wiedergabeeinrichtung zum Wiedergeben von bildhaften Inhalten auf einem Anzeigemittel der Wiedergabeeinrichtung.

Aus der DE 102 33 960 A1 oder aus der US 2009/0156886 A1 ist bekannt, dass auf einem Anzeigemittel, das im Blickfeld einer Person angeordnet ist, wiedergegebener bildhafter Inhalt, also beispielsweise Videos, Fotos, Abfolgen von Fotos, Videoanimationen etc., zum Erzeugen von visuellen Stimuli führt, die sich insbesondere auf den Zustand der Person, beispielsweise auf den Gemütszustand der Person, auswirken können, die mit dem bildhaften Inhalt konfrontiert wird.

Die DE 102 54 051 A1 beschreibt Gerät zur Beeinflussung eines psychischen Zustands und Verfahren zur Erzeugung von sensoriellen Signalen mit Mitteln zur Abgabe von sensoriellen Signalen, Mitteln zur Erfassung eines physiologischen Zustands und Mitteln zur Steuerung der Mittel zur Abgabe von sensoriellen Signalen in Abhängigkeit eines erfassten physiologischen Zustands.

In der US 5,465,729 A bzw. in der US 5,662,117 A ist ein Verfahren und eine Vorrichtung zum Abhalten einer Biofeedbacksitzung für einen Patienten beschrieben. Dabei werden Messungen elektrophysiologischer Größen zur Steuern einer Präsentation einer Serie von audiovisuellen Sequenzen in unterschiedlicher Klarheit bzw. in unterschiedlicher relativer Geschwindigkeit und Audiokomposition verwendet. Die Sequenzen sind reale Szenen, die so konzipiert sind, dass sie bei der Betrachtung einen gewünschten psychologischen Zustand induzieren. Wenn es dem Patienten gelingt, seine physiologischen Parameter zu verändern, verbessert sich die Klarheit bzw. die Geschwindigkeit und Audiokomposition des dargestellten Bildes und Tons als ein Hinweis auf den Erfolg.

Die WO 2012/176098 A1 offenbart ein System, das in der Lage ist, eine Atmosphäre in einem Patientenzimmer zu schaffen und dabei die sensorische Belastung in Abhängigkeit vom Patientenzustand (z. B. Heilungszustand, etwa Schmerzniveau, Erholungsphase oder Fitness) zu dosieren. Der Zustand der Atmosphäre kann aus Sensormessungen bestimmt werden, z.B. Messungen der Körperhaltung des Patienten, seiner Bettposition, seiner Emotionen oder des Ausmaßes seiner körperlichen Aktivität.

Die NL 2 009 753 C bezieht sich auf ein somatisches Signalverarbeitungssystem. Das System besteht aus einem Sensor, der ein somatisches Signal eines Subjekts in ein elektronisches Sensorsignal umwandelt. Das System beinhaltet eine Steuereinheit die mit dem Sensor verbunden sind. Die Steuereinheit beinhaltet einen Prozessor zum Analysieren des Sensorsignals, um Informationen über das Subjekt zu erlangen. Ein mit der Steuereinheit verbundener Aktuator ist ausgebildet, dem Subjekt ein Stimulussignal bereitzustellen, welches von den erlangten Informationen über das Subjekt abhängt.

Die US 5,662,117 A beschreibt ein System und Verfahren zur Beeinflussung eines photobiologischen Zustands eines Wirbeltiers. Das System umfasst eine Lichtquelle zum Aussenden von Licht, das den photobiologischen Zustand beeinflusst, einen Sensor, der angeordnet ist, um einen ersten biophysikalischen Parameter zu erfassen, und eine Steuerschaltung zum Steuern der Lichtquelle, um einen vorbestimmten photobiologischen Zustand zu erzeugen. Die Steuerschaltung empfängt ein Feedbacksignal vom Sensor und sendet ein Steuersignal an die Lichtquelle. Das Steuersignal wird durch Kombination eines zweiten Parameters mit dem ersten biophysikalischen Parameter erzeugt. Der zweite Parameter ist ein zweiter biophysikalischer Parameter oder ein Interaktionsparameter, der eine Interaktion des Wirbeltieres mit einer Vorrichtung charakterisiert. Der zweite Parameter repräsentiert einen weiteren biologischen Zustand des Wirbeltiers.
Aus der WO 2012/104758 A1 ist ein Steuerungssystem für eine Umgebungslichtumgebung in einem Raum in einer Krankenhausumgebung bekannt. Die Steuerung ist konfiguriert, um Lichteffekte der Umgebungslichtumgebung als Reaktion auf ein Sensorsignale von Patientenortungssensoren oder andere Sensoren zeitlich zu steuern und zu synchronisieren.

Die EP 1 656 880 A1 beschreibt ein Bildanzeigesystem, das den Zustand einer Person, die einen PDA trägt, visuell anzeigt. Der PDA beinhaltet einen Biosensor zum Erfassen von Bioinformationen über die Person und einen Umweltinformationssensor, um Informationen über die Umgebung der Person zu erhalten. Der PDA sendet die erfassten Informationen an eine Bildanzeigevorrichtung, die über ein Netzwerk mit dem PDA verbunden ist. Die Bildanzeigevorrichtung beinhaltet eine CPU, die den Zustand der Person auf der Grundlage von Bioinformationen und Umweltinformationen beurteilt und stellt den Zustand als ein Bild auf einem Display dar.

Die DE 102 33 960 A1 offenbart eine Vorrichtung zur bedarfsgesteuerten Modulation physiologischer und pathologischer neuronaler rhythmischer Aktivität im Gehirn mittels sensorischer Stimulation, die in der Lage ist, Funktionsstörungen des Gehirns diagnostisch zu sichern sowie die Symptomatik der Funktionsstörung zu mildern oder aufzuheben. Die Vorrichtung umfasst eine Steuereinheit, einen Stimulator sowie mindestens ein Mittel zur Erfassung der Hirnaktivität, welches mit der Steuereinheit in Verbindung steht.

### Beschreibung

Es kann wünschenswert sein, das Erzeugen derartiger visueller Stimuli in vorteilhafter Weise automatisiert und deterministisch erfolgen zu lassen.

Erfindungsgemäß werden die Gegenstände der unabhängigen Patentansprüche vorgeschlagen. Merkmale einiger Ausführungsformen sind in den Unteransprüchen angegeben.

Einen ersten Aspekt bildet eine Steuereinrichtung zum Steuern einer Wiedergabeeinrichtung. Die Steuereinrichtung ist ausgebildet zum Wiedergeben von bildhaften Inhalt auf einem Anzeigemittel der Wiedergabeeinrichtung, um visuelle Stimuli für eine Person zu erzeugen. Die Steuereinrichtung umfasst: eine Erfassungseinrichtung, die ausgebildet ist zum Erfassen von personenbezogenen Daten, die indikativ für wenigstens ein Symptom der Person sind, und zum Bereitstellen eines Erfassungsergebnisses in Abhängigkeit von den personenbezogenen Daten, wobei die Erfassungseinrichtung zum Erfassen der personenbezogenen Daten aufweist: eine Sensoreinrichtung, die ausgestaltet ist für eine Ankopplung an die Person, um personenbezogenen Daten in Gestalt von Sensorinformationen über das wenigstens eine Symptom der Person zu ermitteln, wobei die Sensoreinrichtung eine Vielzahl von Sensoren aufweist, wobei die Sensorinformationen über das wenigstens eine Symptom Werte von Körpermessgrößen umfassen, wobei jeder der Sensoren ausgebildet ist zum Bestimmen eines Werts einer bestimmten Körpermessgröße; einen Controller, der ausgebildet ist zum Bestimmen wenigstens eines Werts eines Wiedergabeparametersatzes in Abhängigkeit von dem Erfassungsergebnis, wobei der Controller weiter ausgebildet ist, eine Anzahl der Vielzahl von Sensoren auszuwählen in Abhängigkeit von den Sensorinformationen, um Werte einer Anzahl ausgewählter Körpermessgrößen zu bestimmen; und die Erfassungseinrichtung ausgebildet ist, das Erfassungsergebnis in Abhängigkeit von den bestimmten Werten bereitzustellen; und eine Steuereinheit, die ausgebildet ist zum Steuern der Wiedergabe eines in Abhängigkeit von dem wertmäßig bestimmten Wiedergabeparametersatz parametrisierten bildhaften Inhalts.

Einen zweiten Aspekt bildet ein Computerprogramm zum Steuern einer Wiedergabeeinrichtung, die ausgebildet ist zum Wiedergeben von bildhaften Inhalt auf einem Anzeigemittel der Wiedergabeeinrichtung, um visuelle Stimuli für eine Person zu erzeugen. Das Computerprogramm ist ausgebildet eine Steuereinrichtung zu veranlassen, ein Verfahren zum Steuern der Wiedergabeeinrichtung durchzuführen. Das Verfahren umfasst: Erfassen von personenbezogenen Daten, die indikativ für wenigstens ein Symptom der Person sind, und Bereitstellen eines Erfassungsergebnisses in Abhängigkeit von den personenbezogenen Daten, wobei das Erfassen der personenbezogenen Daten über eine Sensoreinrichtung erfolgt, die ausgestaltet ist für eine Ankopplung an die Person, um personenbezogenen Daten in Gestalt von Sensorinformationen über das wenigstens eine Symptom der Person zu ermitteln, wobei die Sensoreinrichtung eine Vielzahl von Sensoren aufweist, wobei die Sensorinformationen über das wenigstens eine Symptom Werte von Körpermessgrößen umfassen, wobei jeder der Sensoren ausgebildet ist zum Bestimmen eines Werts einer bestimmten Körpermessgröße; Bestimmen wenigstens eines Werts eines Wiedergabeparametersatzes in Abhängigkeit von dem Erfassungsergebnis, wobei das Bestimmen ein Auswählen einer Anzahl der Vielzahl von Sensoren in Abhängigkeit von den Sensorinformationen umfasst, um Werte einer Anzahl ausgewählter Körpermessgrößen zu bestimmen; Bereitstellen des Erfassungsergebnisses in Abhängigkeit von den bestimmten Werten; und Steuern der Wiedergabe eines in Abhängigkeit von dem wertmäßig bestimmten Wiedergabeparametersatz parametrisierten bildhaften Inhalts.

### Kurze Figurenbeschreibung

Der der Erfindung zugrunde liegende Gedanke soll nachfolgend anhand der in den Figuren dargestellten Ausführungsbeispiele näher erläutert werden. Darin zeigen
- Fig. 1: eine schematische und exemplarische Darstellung einer Ausführungsform einer Steuereinrichtung;
- Fig. 2: eine schematische und exemplarische Darstellung von bildhaften Inhalt, der in Abhängigkeit der Tageszeit parametrisiert worden ist;
- Fig. 3: eine schematische und exemplarische Darstellung eines Diagramms zur Veranschaulichung einer Parametrisierung von bildhaften Inhalt; und
- Fig. 4: eine schematische und exemplarische Darstellung eines Flussdiagramms zur Veranschaulichung einer Auswahl von Sensoren der Sensormesseinrichtung.

### Ausführliche Beschreibung

Die Steuereinrichtung dient zum Steuern einer Wiedergabeeinrichtung, die ausgebildet ist zum Wiedergeben von bildhaften Inhalt auf einem Anzeigemittel der Wiedergabeeinrichtung, um visuelle Stimuli für eine Person zu erzeugen.

Die Steuereinrichtung umfasst eine Erfassungseinrichtung, die ausgebildet ist zum Erfassen von personenbezogenen Daten, die indikativ für wenigstens ein Symptom der Person sind. Die Erfassungseinrichtung ist ferner ausgebildet zum Bereitstellen eines Erfassungsergebnisses in Abhängigkeit von den erfassten personenbezogenen Daten.

An die Erfassungseinrichtung gekoppelt, beispielsweise nachgeschaltet, ist ein Controller, der das Erfassungsergebnis empfängt und ausgebildet ist zum Bestimmen wenigstens eines Werts eines Wiedergabeparametersatzes in Abhängigkeit von dem Erfassungsergebnis.

Außerdem umfasst die Steuereinrichtung eine Steuereinheit, die ausgebildet ist zum Steuern der Wiedergabe eines in Abhängigkeit von dem wertmäßig bestimmten Wiedergabeparametersatz parametrisierten bildhaften Inhalts. Die Steuereinheit gibt den parametrisierten bildhaften Inhalt auf dem Anzeigemittel aus.

In ihrer Gemeinsamkeit sind der Controller und die Steuereinheit demnach beispielsweise ausgebildet, das Erfassungsergebnis in den parametrisierten bildhaften Inhalt zu transformieren, der dann von der Steuereinheit auf dem Anzeigemittel der Wiedergabeeinrichtung wiedergegeben werden kann.

Die vorliegende Erfindung geht von den folgenden Erkenntnissen aus und umfasst die folgenden Grundgedanken:
Eine Person, die beispielsweise wegen einer Erkrankung und/oder einer Verletzung zu behandeln ist, also beispielsweise stationär in einem Krankenhaus behandelt wird, zeigt häufig eine bestimmte Anzahl unerwünschter Symptome. Dabei treten manche Symptome häufiger auf als andere, wobei bestimmte Symptome den Heilungsprozess bei der Person unterschiedlich stark beeinflussen können. Zur Linderung derartiger Symptome oder Syndrome werden häufig Medikamente eingenommen, die aber wiederum zu anderen unerwünschten Nebenwirkungen und/oder Symptomen führen. Daher kann es zweckmäßig sein, nicht-pharmakologische Maßnahmen den pharmakologischen Maßnahmen zur Heilung der Person vorzuziehen, wenigstens aber additiv in der Therapie oder Prävention von Symptome einzusetzen, um die Medikamentendosis zu reduzieren.

Die vorliegende Erfindung geht mitunter davon aus, dass visuelle Stimuli zu derartigen nicht-pharmakologischen Maßnahmen gehören.

Bei den bekannten Gegenständen zum Erzeugen derartiger visueller Stimuli, wie sie beispielsweise in den Literaturstellen [1] bis [9] beschrieben sind, ist jedoch problematisch, dass diese Gegenstände allenfalls eine geringe Flexibilität bei der Erzeugung der visuellen Stimuli erlauben und eine Anpassung des bildhaften Inhalts an unterschiedliche Symptome bzw. Symptomkonstellationen (im Folgenden auch als Syndrome bezeichnet) oder an Schweregrade von Symptomen nicht möglich sind. Dieser Aspekt ist jedoch insbesondere im intensiv-stationären Kontext von hoher Relevanz. Kritisch kranke Patienten haben nämlich meist eine Vielzahl von Symptomen unterschiedlicher Schwere, die gleichzeitig auftreten und so individuelle Syndrome ausbilden. Dabei kann es bei bestimmten Syndromen, insbesondere mehrmals täglich, zur signifikanten Fluktuationen der Symptomkonstellationen kommen.

Die bekannten Gegenstände zum Erzeugen visueller Stimuli sind nicht ausgebildet, den bildhaften Inhalt an die gerade beschriebenen Fluktuationen anzupassen. Mit den bisher bekannten technischen Lösungen ist somit eine unmittelbare Anpassung visueller Inhalte an verändernde Symptomkonstellationen nicht möglich. Dies führt dazu, dass das Anzeigen des nicht angepassten bildhaften Inhalts visuelle Stimuli produziert, die für den Heilungsprozess nicht nur nicht wirksam sein können, sondern auch negative Auswirkungen haben können.

Im Gegensatz dazu gibt eine Ausführungsform der Steuereinrichtung beispielsweise parametrisierten bildhaften Inhalt aus, welcher das wenigstens eine Symptom der Person, z.B. eine Symptomkomplexität, eine Symptomfluktuation und/oder einen Schweregrad aktuell vorliegender Symptome bei der Person, berücksichtigt, sodass eine Anpassung des bildhaften Inhalts, sprich die Parametrisierung des bildhaften Inhalts, an die aktuelle Situation der Person quasi in Echtzeit und z.B. automatisch und deterministisch erfolgen kann. Die Parametrisierung des bildhaften Inhalts durch den Controller erfolgt also beispielsweise zum einen nicht in zufälliger Weise, sondern in deterministischer Weise in Abhängigkeit von dem Erfassungsergebnis, und zum anderen mit möglichst geringer Zeitverzögerung.

Beispielsweise ist die Steuereinrichtung ausgebildet, manuell eingegebene und/oder selbsttätig erfasste personenbezogenen Daten zu verarbeiten und aus diesen Daten Bewegtbilder, also den bildhaften Inhalt zur Symptom-Therapie und/oder zur Symptom-Prävention, zu generieren, die auf einer darstellenden Hardware, nämlich dem Anzeigemittel der Wiedergabeeinrichtung, zu übertragen und dort darzustellen sind.

Beispielsweise bilden die manuell eingegebenen bzw. selbsttätig erfassten personenbezogenen Daten eine Testreihe und/oder eine Trainingseinheit ab. Die medizinische Testreihe wird beispielsweise durch den Controller in eine aufeinander aufbauende, logische Reihenfolge gebracht, welche sowohl die Durchführung der Tests verbessert als auch die Fehlermöglichkeit bei der Erfassung verringert. Die beispielsweise vom Controller gesteuerte Trainingseinheit erlaubt es beispielsweise der Person, ihre kognitiven Fähigkeiten automatisiert oder manuell zu trainieren.

Wie weiter unten näher erläutert ist, ist die Erfassungseinrichtung nach einer Ausführungsform ausgebildet, nicht nur personenbezogene Daten zu erfassen, sondern auch Umgebungsdaten, beispielsweise Tageszeit- und/oder Wetter- und/oder Positionsinformationen, die die Steuereinrichtung z.B. dazu nutzt, um der Person mittels der visuellen Stimuli Orientierungshilfe zu bieten. Ebenso ist es bei Ausführungsformen möglich, dass die manuell eingegebenen bzw. selbsttätig erfassten personenbezogenen Daten eine Basis für therapeutische Sessions (Sitzungen) bilden, die je nach Therapieart ein visuelles Biofeedback ausgeben können, was an späterer Stelle ebenfalls näher erläutert werden wird.

Bei der Steuereinrichtung übernimmt beispielsweise eine Benutzerschnittstelle die Rolle einer Eingabeoberfläche, über die zum die personenbezogenen Daten eingegeben werden und/oder automatisch erfasst werden können und mit der ein Benutzer der Steuereinrichtung durch Eingabe entsprechender Benutzereingaben Einfluss auf die Wiedergabe des parametrisierten bildhaften Inhalts nehmen kann. Der Controller der Steuereinrichtung übernimmt beispielsweise die Rolle einer Art Middleware, der sämtliche erfassten Daten in Gestalt des Erfassungsergebnisses und ggf. Benutzereingaben empfängt, auswertet und in Abhängigkeit von der Auswertung den Wiedergabeparametersatz wertmäßig bestimmt und diesen der Steuereinheit bereitstellt. Die Steuereinheit der Steuereinrichtung übernimmt schließlich eine Art Renderer, der den in Abhängigkeit von dem Wiedergabeparametersatz parametrisierten bildhaften Inhalt auf dem Anzeigemittel der Wiedergabeeinrichtung ausgibt.

Das Anzeigemittel in Blickrichtung der Patienten wird beispielsweise unter Verwendung eines parametrischen Designs mit unterschiedlichen Schichten (Layern) moduliert.

Statt beispielsweise unterschiedliche Szenen für unterschiedliche Symptome oder Syndrome zu verwenden, erzeugt die Steuereinrichtung beispielsweise eine zusammengesetzte dynamische Szene, deren Einzelbestandteile entsprechend der vorliegenden Symptome und/oder Syndrome und ihrer jeweiligen Stärke sowie weiterer kontextueller Daten gewichtet werden können.

Die Steuereinrichtung dient somit beispielsweise zum Steuern eines Anzeigemittels in Gestalt einer stimulierenden Leinwand, die auf den Zustand der Person abgestimmte, adaptive kognitive Anregungen in Gestalt der visuellen Stimuli liefert und die Genesung der Person unterstützen soll.

Nachfolgend sollen optionale Komponenten und einige optionale Aspekte der Steuereinrichtung näher beschrieben werden:

### Wiedergabeeinrichtung mit Anzeigemittel

Das Anzeigemittel der Wiedergabeeinrichtung umfasst beispielsweise einen großformatigen, medial bespielbarer Screen, der über einem Bett angebracht werden kann, bspw. an der Zimmerdecke, und/oder beispielsweise für eine Befestigung an einer Wand ausgebildet ist.

Das Anzeigemittel umfasst beispielsweise ein LED-Raster, das durch die Steuereinrichtung, z.B. durch die Steuereinheit, über ein DMXKiNet-Protokoll angesteuert werden kann. Die Größe und die Auflösung des Anzeigemittels sind beispielsweise variabel. Beispielsweise können die LEDs des Anzeigemittels sämtliche Farben im RGB-Farbraum wiedergeben.

Beispielsweise umfasst die Steuereinrichtung ferner einen Lichtsensor, der ausgebildet ist zum Bestimmen einer Lichtstärke und/oder einer Lichttemperatur (auch als Farbtemperatur bezeichnet) eines Umgebungslichts, welches die Person umgibt, sodass die Steuereinheit die Helligkeit des Anzeigemittels und/oder anderer Darstellungsparameter in Abhängigkeit von dem Umgebungslicht anpassen kann.

Es ist ferner möglich, dass die Wiedergabeeinrichtung neben dem Anzeigemittel eine Beleuchtungseinrichtung aufweist, die zum Beleuchten eines Zimmers ausgestaltet ist. Die Steuereinrichtung kann ausgebildet sein, die Beleuchtungseinrichtung basierend auf dem Wiedergabeparametersatz zu steuern, also beispielsweise in Abhängigkeit von der Lichtstärke und/oder Lichttemperatur des Umgebungslichts. Für diese Zwecke erfolgt die Steuerung der Beleuchtungseinrichtung beispielsweise nach Maßgabe des DALI (Digital Addressable Lighting Interface) oder des DMX (Digital Multiplex) Protokolls.

Bei einer Ausführungsform ist die Steuereinrichtung ausgebildet ist, in Abhängigkeit von dem Erfassungsergebnis wahlweise in einem der folgenden Modi zu operieren:
- einen Normalmodus, bei dem die visuellen Stimuli in Abhängigkeit von dem Erfassungsergebnis erzeugt werden;
- einen Notfallmoduls, bei dem die Erzeugung der visuellen Stimuli ausgesetzt wird.

Im Normalmodus erfolgt die Erzeugung der visuellen Stimuli, beispielsweise, wie es oben dargestellt worden ist. Erkennt der Controller jedoch beispielsweise, dass das Erfassungsergebnis indikativ für einen kritischen Zustand der Person (wie eine Herzinsuffizienz) ist, so wird die Erzeugung der visuellen Stimuli ausgesetzt. Beispielsweise bedient die Steuereinrichtung dann die Beleuchtungseinrichtung derart, dass die Person passierendes Personal auf den kritischen Zustand der Person aufmerksam gemacht wird. Denkbar ist beispielsweise, dass die Steuereinrichtung die Beleuchtungseinrichtung derart bedient, dass diese ein Warnlicht emittiert oder ähnliches.

Bei einer weiteren Ausführungsform ist die Steuereinrichtung ausgebildet, die Beleuchtungseinrichtung gemäß einem circadianen Rhythmus zu steuern. Auf diese Weise kann ein Schlaf-Wach-Rhythmus der Person unterstützt werden.

Die Steuereinrichtung ist beispielsweise drahtgebunden oder drahtlos an das Anzeigemittel und ggf. an die Beleuchtungseinrichtung der Wiedergabeeinrichtung gekoppelt. Aufgrund dieser Kopplung kann die Steuereinheit Daten, die den parametrisierten bildhaften Inhalt enthalten, an das Anzeigemittel übermitteln und dort ausgeben, bzw. Steuersignale an die Beleuchtungseinrichtung übermitteln.

### Bildhafter Inhalt und visuelle Stimuli

Bei dem bildhaften Inhalt handelt es sich beispielsweise um Bewegtbilder, die durch die Steuereinrichtung automatisch und in Echtzeit parametrisiert werden. Der bildhafte Inhalt umfasst beispielsweise ein Stehbild, eine Abfolge von Stehbildern, Bewegtbilder, Videoanimationen, Videos und/oder Audioinhalte und/oder sonstige Contentelemente. Daten, die derartigen bildhaften Inhalt enthalten, werden von der Steuereinrichtung parametrisiert und zum Anzeigemittel übermittelt, um dort visuelle bzw. audiovisuelle Stimuli zu erzeugen. Konkrete Beispiele für den bildhaften Inhalt werden im Folgenden stichpunktartig angegeben:
- ein Sternenhimmel, der beispielsweise langsam bewegt durch das Anzeigemittel dargestellt wird und für den der Wiedergabeparametersatz beispielsweise angibt, wie schnell sich die Sterne bewegen, wie viele Sterne angezeigt werden, eine Dichteverteilung etc.
- ein Himmelshintergrund mit einer Sonne, wobei der Wiedergabeparametersatz beispielsweise in Abhängigkeit von der Tageszeit und/oder von der geografischen Position der Person bestimmt, an welcher Stelle die Sonne darzustellen ist, wie groß und hell sie ist, und wie die Himmelsfarbe ausgestaltet ist, etc.
- Wolken, für die der Wiedergabeparametersatz beispielsweise angibt, wie schnell sie zu bewegen sind, welche Dimension, Ausgestaltung und welche Farbe sie haben sollen. Der Wiedergabeparametersatz wird dabei beispielsweise in Abhängigkeit von aktuellen tatsächlichen Wetterdaten bestimmt, die von der Erfassungseinrichtung zuvor ermittelt worden sind.
- Blattwerk, beispielsweise eine Anzahl von Laubblättern, wobei die Dichte der Blätter, die Skalierung und/oder die Bewegung der Blätter durch den Wiedergabeparametersatz vorgegeben werden.
- eine zwei- oder mehrfarbige Lichtfläche, wobei die Geschwindigkeit und die Richtung von Farbwechseln auf dem Anzeigemittel durch den Wiedergabeparametersatz vorgegeben werden.
- bewegende Lichtpunkte, wobei eine Geschwindigkeit der Bewegungen sowie eine Dichte der bewegenden Lichtpunkte auf dem Anzeigemittel durch den Wiedergabeparametersatz vorgegeben werden.

Der parametrisierte bildhafte Inhalt umfasst beispielsweise passive Inhalte und/oder aktive Inhalte.

Bei den passiven Inhalten handelt es sich beispielsweise um einen parametrisierten bildhaften Inhalt, der automatisiert abläuft und ein aktives Zutun der Person nicht erfordert. Für die Parametrisierung des passiven bildhaften Inhalts führt beispielsweise ein Klinikpersonal mittels der Erfassungseinrichtung eine Messreihe an der Person durch, sodass der Controller, beispielsweise basierend auf einen deterministischen Algorithmus, in Abhängigkeit von dem Erfassungsergebnis den Wiedergabeparametersatz bestimmen kann, basierend auf dem die Parametrisierung des bildhaften Inhalts erfolgt. Allerdings kann gemäß einem anderen Ausführungsbeispiel die durch Durchführung der Messreihe allein durch die Steuereinrichtung erfolgen, z.B. also ohne Personal.

Bei den aktiven Inhalten handelt es sich beispielsweise um visuelle Inhalte, mit denen die Person in aktive Interaktion treten kann. Die Person kann beispielsweise die Parametrisierung des bildhaften Inhalts durch bestimmte Aktionen, wie Bewegungen von Körperteilen, Bewegung der Augen/Augenlieder etc., die durch die Erfassungseinrichtung erfasst werden, beeinflussen. Auf diese Weise können mittels der Steuereinrichtung beispielsweise therapeutischen Sessions mit der Person durchgeführt werden. Beispielsweise können kognitive Fertigkeiten trainiert werden, es kann eine Unterstützung einer Physiotherapie und/oder einer Mobilisation erfolgen, und/oder es erfolgt eine Unterstützung bei einer Beatmungstherapie und/oder eine Beatmungsentwöhnung.

Jedenfalls erzeugt die Steuereinrichtung nach einer Ausführungsform für unterschiedliche Symptome/Syndrome unterschiedlichen bildhaften Inhalt. Da der Wiedergabeparametersatz beispielsweise auf Basis kontinuierlicher Auswertungen der personenbezogenen Daten aktualisiert wird, passt sich der bildhafte Inhalt an den aktuellen Zustand der Person an. Die Steuereinrichtung ermöglicht es also, der Person einen kontinuierlichen, wiederholungsfreien bildhaften Inhalt zu präsentieren.

### Person

Die Person ist beispielsweise eine immobile Person, wie ein Patient, der zur stationären Behandlung in einem Patientenzimmer eines Krankenhauses liegt. Beispielsweise handelt es sich bei der Person um eine Person, die sich in einem Aufwachprozess aus einem Koma oder komaähnlichen Zustand befindet. Jedenfalls weist die Person einen physischen und psychischen Zustand auf, der zeitabhängig ist. Dieser Zustand der Person wird durch Erfassung der personenbezogenen Daten ermittelt. Die personenbezogenen Daten sind beispielsweise indikativ für wenigstens eine der folgenden personenbezogenen Symptome und/oder Syndrome: Agitation, Angst, Delir, Desorientierung, Halluzination, Schmerz und/oder Sedierung. Derartige Symptome können in verschiedenen Kombinationen und in verschiedenen Stärken auftreten. In Abhängigkeit von der Symptomkombination und/oder in Abhängigkeit von der Stärke der einzelnen Symptome erfolgt die Parametrisierung des bildhaften Inhalts.

### Erfassungseinrichtung, Benutzerschnittstelle und personenbezogene Daten

Allgemein gesprochen dient die Erfassungseinrichtung zum Erfassen sämtlicher personenbezogener Daten, die den Zustand der Person charakterisieren, z.B. zum Erfassen des wenigstens einen Symptoms, das die Person aufweist. Darüber hinaus kann die Erfassungseinrichtung zum Erfassen von Umgebungsdaten dienen, die die Umgebung der Person charakterisieren, wie bspw. die aktuelle Uhrzeit, die aktuelle Position, das aktuelle Wetter, die aktuellen Lichtverhältnisse etc.

Die Steuereinrichtung umfasst zum Erfassen der personenbezogenen Daten beispielsweise ferner eine Benutzerschnittstelle, die ausgebildet ist zum Empfangen von personenbezogenen Daten in Gestalt von Benutzereingaben eines Benutzers der Steuereinrichtung, wobei die Benutzereingaben das wenigstens eine Symptom der Person betreffen.

Darüber hinaus kann die Erfassungseinrichtung beispielsweise eine Sensoreinrichtung umfassen, die ausgestaltet ist für eine Ankopplung an die Person, um personenbezogene Daten in Gestalt von Sensorinformationen über das wenigstens eine Symptom der Person zu ermitteln.

Mit anderen Worten können die personenbezogenen Daten manuell eingegeben werden mittels der Benutzerschnittstelle. Die Erfassungsvorrichtung kann darüber hinaus oder alternativ dazu die personenbezogenen Daten aber auch mittels der Sensoreinrichtung erfassen.

Die Benutzerschnittstelle ist beispielsweise an ein Eingabegerät der Steuereinrichtung gekoppelt, das zum Produzieren und Aussenden der Benutzereingaben ausgebildet ist. Auf diese Weise kann die Steuereinrichtung die Benutzereingaben drahtlos empfangen. Mittels des Eingabegeräts kann ein Benutzer - beispielsweise die Person selbst oder die Person betreuendes Personal - der Steuereinrichtung beispielsweise identifizierte klinische Symptome mittels einer grafischen Oberfläche eingeben bzw. mittels des Eingabegeräts eine Messreihe oder Befragung durchführen, die zur Ermittlung einer aktuell bei der Person vorliegenden Symptomkonstellation dient. Die Benutzereingaben können nicht nur Symptome oder Syndrome der Person betreffen, sondern auch Steueranweisungen, die die Steuereinrichtung bei der Wiedergabe des parametrisierten bildhaften Inhalts zu berücksichtigen hat.

Beispielsweise ist die Erfassungseinrichtung darüber hinaus an ein Patientendatenmanagementsystem (PDMS) gekoppelt, um weitere Daten über die Person zu erfassen. Mittels der Ankopplung an das PDMS kann die Erfassungseinrichtung in einer individuellen Patientenakte, die der Person zugeordnet ist, weitere personenbezogenen Daten, wie beispielsweise historische Patienteninformationen, erfassen.

Beispielsweise ist das bereits angesprochene Eingabegerät ausgebildet, eine standardisierte medizinische Testreihe durchzuführen, um Symptome und/oder Syndrome bei der Person zu ermitteln. Dies erfolgt beispielsweise im Rahmen einer Befragung der Person.

Bei einer weiteren Ausführungsform ist der Controller ausgebildet, eine Ausführung eines Programms auf dem Eingabegerät zu steuern in Abhängigkeit von den Benutzereingaben und/oder in Abhängigkeit von dem Erfassungsergebnis. Beispielsweise sendet der Controller über die Benutzerschnittstelle Steuerbefehle an das Eingabegerät, um die Ausführung des Programms zu steuern, beispielsweise, um also eine nächste Frage eines Befragungsschemas zu bestimmen oder einen einzusetzenden Sensor zu bestimmen, der im Rahmen einer Messreihe zu bedienen ist.

Bei dem Programm, das auf dem Eingabegerät durchgeführt wird, handelt es sich beispielsweise um ein Befragungsschema (Anamnese-Schema) oder ein Diagnoseschema, gemäß dem die Person von einem Personal befragt bzw. diagnostiziert wird. Antworten auf Fragen oder Diagnoseergebnisse werden in das Eingabegerät eingegeben und von dem Eingabegeräte als Benutzereingaben an die Steuereinrichtung übertragen. Dadurch, dass die Steuerung des Programms durch die Steuereinrichtung in Abhängigkeit von den Benutzereingaben und/oder in Abhängigkeit von dem Erfassungsergebnis erfolgt, also beispielsweis in Abhängigkeit von den Sensorinformationen, erfolgt die Befragung bzw. die Diagnose nicht linear, sondern situativ.

Beispielsweise handelt es sich bei dem Eingabegerät um ein mobiles Endgerät.

Die in das Eingabegerät eingegebenen Daten überträgt das Eingabegerät beispielsweise drahtlos über WLAN oder gemäß einem sonstigen Drahtloskommunikationsstandard als Benutzereingaben an die Steuereinrichtung. In Abhängigkeit der empfangenen Benutzereingaben und/oder in Abhängigkeit des Erfassungsergebnisses kann die Steuereinrichtung auch Befehle an das Eingabegerät zurücksenden, so dass basierend auf diesen Befehlen eine Testreihe fortgeführt werden kann, sofern sie noch nicht abgeschlossen worden ist.

Beispielsweise sind die Steuereinrichtung und das Eingabegerät also ausgebildet, drahtlos miteinander zu kommunizieren.

Beispielsweise ist das Eingabegerät ausgebildet, die Benutzereingaben in einem vordefinierten Format an die Steuereinrichtung zu übertragen.

Nach einer Ausführungsform erlaubt die Benutzerschnittstelle, die optional Teil der Steuereinrichtung ist, eine manuelle Eingabe von personenbezogenen Daten in Gestalt Benutzereingaben und die Ausgabe von Befehlen an das Eingabegerät. Auf diese Weise kann ein Benutzer der Steuereinrichtung, beispielsweise ein Arzt oder ein Pflegepersonal, und/oder die Person selbst, Einfluss auf die Parametrisierung des bildhaften Inhalts nehmen.

Zum selbsttätigen Erfassen der personenbezogenen Daten kann die Erfassungseinrichtung ferner die bereits erwähnte Sensoreinrichtung mit einer Anzahl von Sensoren aufweisen, um Sensorinformationen über das wenigstens eine Symptom der Person zu ermitteln. Beispielsweise umfasst die Sensoreinrichtung ein oder mehrere Sensoren, beispielsweise validierte Messinstrumente, um Symptome wie Agitation, Angst, Delir, Desorientierung, Halluzination, Schmerz und/oder Sedierung zu ermitteln.

Die Sensorinformationen über das wenigstens eine Symptom umfassen beispielsweise Werte von Körpermessgrößen. Bei der Körpermessgrößen handelt es sich also um messbare Größen, die zur Bestimmung eines oder mehrerer der gerade genannten Symptome geeignet sind. Die Körpermessgrößen umfassen beispielsweise wenigstens eines des Folgenden: eine Körpertemperatur der Person, einen Blutdruck der Person, eine oder mehrere Messgrößen, die im Rahmen einer bei der Person durchgeführten Elektroenzephalografie ermittelt worden sind, eine Herzschlagrate, eine Atemfrequenz, etc.

Umfasst die Sensoreinrichtung beispielsweise eine Vielzahl von Sensoren, von denen jeder ausgebildet ist zum Bestimmen eines Werts einer bestimmten Körpermessgröße, so ist der Controller z.B. ausgebildet, eine Anzahl der Vielzahl von Sensoren auszuwählen, z.B. in Abhängigkeit von den empfangenen Benutzereingaben und/oder von dem Erfassungsergebnis, um Werte einer Anzahl ausgewählter Körpermessgrößen zu bestimmen, und wobei die Erfassungseinrichtung ausgebildet ist, das Erfassungsergebnis in Abhängigkeit von den bestimmten Werten bereitzustellen.

Der Controller ist nach einer Ausführungsform ausgebildet, eine Reihenfolge der einzusetzenden Sensoren der Sensoreinrichtung in Abhängigkeit von den Benutzereingaben und/oder in Abhängigkeit von den Sensorinformationen zu bestimmen. Beispielsweise ermittelt der Controller basierend auf den Benutzereingaben und/oder basierend auf dem Erfassungsergebnis, welcher Sensor/welches Messinstrument der Sensoreinrichtung wann einzusetzen ist, um weitere personenbezogene Daten zu ermitteln.

Beispielsweise erfolgt eine Eingabe von Messergebnissen über eine hierfür vorgesehene Controller-App, die von dem Eingabegerät ausgeführt wird. Abhängigkeiten der einzelnen Messergebnisse innerhalb einer an der Person durchgeführten Testreihe werden dabei berücksichtigt und interpretiert. Beispielsweise können Schmerzen bei einer deliranten Person nur mittels eines bestimmten Sensors/eines bestimmten Messinstruments erfasst werden. Anderenfalls, also bei einer nicht deliranten Person, wählt der Controller beispielsweise generell subjektive Schmerzmessinstrumente für die Erfassung weiterer personenbezogener Daten selbsttätig aus.

Nach einer Ausführungsform ist die Erfassungseinrichtung ausgebildet, sämtliche Daten, die die Person sowie die diese umgebende Umgebung betreffen, zu erfassen, und zwar selbsttätig und/oder basierend auf über die Benutzerschnittstelle eingegebenen Benutzereingaben, wobei die Erfassungseinrichtung darüber hinaus ausgebildet sein kann, die zum Erfassen der personenbezogenen Daten und/oder Umgebungsdaten zweckmäßigen Sensoren/Messinstrumente basierend auf zuvor erfassten Daten und/oder Benutzereingaben selbsttätig auszuwählen und zum Erfassen der Daten zu betreiben. Die von der Erfassungseinrichtung erfassten Daten stellt die Erfassungseinrichtung beispielsweise gebündelt in dem Erfassungsergebnis dar, das dem Controllerzugeführt ist.

### Lichtsensor, Empfänger für Wetterdaten/Positionsdaten

Die Erfassungseinrichtung kann - wie gesagt -zum Erfassen von Umgebungsdaten und zum Bereitstellen des Erfassungsergebnisses auch in Abhängigkeit von den Umgebungsdaten ausgebildet sein. Für diese Zwecke umfasst die Erfassungseinrichtung beispielsweise besagten Lichtsensor, besagten Empfänger für Wetterdaten und/oder Positionsdaten und/oder besagte Zeitmesseinrichtung.

Der Lichtsensor ist ausgebildet zum Bestimmen einer Lichtstärke und/oder einer Lichttemperatur eines Umgebungslichts, das die Person umgibt. Dies erlaubt eine Anpassung der Wiedergabe des parametrisierten bildhaften Inhalts an die Lichtstärke und/oder die Lichttemperatur des Umgebungslichts.

Zum einen wird also beispielsweise der Tatsache Rechnung getragen, dass der Kontrast bei hoher Lichtstärke erhöht werden sollte, sodass die Person den bildhaften Inhalt auch bei hoher Lichtstärke gut erkennen kann, und zum anderen der Tatsache, dass das Anzeigemittel bei der Wiedergabe des parametrisierten bildhaften Inhalts ebenfalls eine Lichtquelle darstellt, die ggf. maximale oder minimale Lichtstärken berücksichtigen muss, mit denen die Person konfrontiert werden darf. Eine Anpassung der Lichttemperatur durch entsprechende Steuerung des Anzeigemittels kann in ähnlicher Weise erfolgen.

Darüber hinaus kann die Wiedergabeeinrichtung neben dem Anzeigemittel auch besagte Beleuchtungseinrichtung umfassen und die Steuereinrichtung ist beispielsweise ausgebildet, die Beleuchtungseinrichtung in Abhängigkeit von dem Wiedergabeparametersatz, also beispielsweise in Abhängigkeit von der Lichtstärke und/oder der Lichttemperatur des Umgebungslichts, zu steuern.

Der Empfänger ist ausgebildet zum Empfangen von Wetterdaten und/oder Positionsdaten, die indikativ für das aktuelle und/oder zukünftige Wetter in der Umgebung der Person sind bzw. indikativ für den Aufenthaltsort der Person sind. Beispielsweise ist der Empfänger der Erfassungseinrichtung dazu an das Internet gekoppelt, um die Wetterdaten und/oder die Positionsdaten zu ermitteln. Die Wetterdaten und/oder die Positionsdaten könnten aber auch selbsttätig durch den Empfänger ermittelt werden, beispielsweise durch entsprechend ausgestaltete Komponenten wie einen GPS-Empfänger und/oder eine Wetterstation.

Die optional vorgesehene Zeitmesseinrichtung ist ausgebildet zum Bereitstellen von Zeitdaten, die indikativ für die aktuelle Uhrzeit sind.

Aufgrund der Wetterdaten und/oder der Positionsdaten und/oder der Zeitdaten ist eine weitere Anpassung, also eine genauere Parametrisierung des wiederzugebenen bildhaften Inhalts an die aktuelle Umgebung der Person möglich.

Beispielsweise umfasst der parametrisierte bildhafte Inhalt eine aufgehende Sonne bei klarem Himmel, sofern die Zeitdaten indikativ für einen Morgen sind und die Wetterdaten gutes Wetter anzeigen. Gleiches gilt sinngemäß für Abendstunden, zu denen der parametrisierte bildhafte Inhalt beispielsweise eine untergehende Sonne vor dichteren Wolken umfasst, sofern die Wetterdaten auf Bewölkung hindeuten. Derartige Beispiele ließen sich weiter fortsetzen.

### Erfassungsergebnis

Das von der Erfassungseinrichtung bereitgestellte Erfassungsergebnis liegt beispielsweise in Gestalt eines Datensatzes vor, der beispielsweise die - ggf. verarbeiteten - personenbezogenen Daten und optional zusätzlich die - ggf. verarbeiteten - Umgebungsdaten umfasst. Das Erfassungsergebnis ist also beispielsweise in einem Datensatz enthalten, der die bei der Person aktuell auftretenden Symptome sowie die Umgebung der Person beschreibt.

Basierend auf einen derartigen Datensatz, der die aktuelle Situation der Person sowie die Umgebung der Person beschreibt, erfolgt die Bestimmung des Wiedergabeparametersatzes durch den Controller.

### Controller

Der Controller bestimmt aufgrund des Erfassungsergebnisses und ggf. aufgrund der Benutzereingaben, wie der wiederzugebene bildhafte Inhalt zu parametrisieren ist.

Der Controller liefert eine Anpassung des bildhaften Inhalts zum einen an die aktuell bei der Person vorliegenden Symptome und zum anderen - optional - an die Umgebung der Person. Die Art und Weise der Parametrisierung wird durch den bestimmten Wiedergabeparametersatz festgelegt. Dieser Wiedergabeparametersatz enthält beispielsweise eine Anzahl von Wiedergabeparametern, wie beispielsweise einen Wiedergabeinhalt, eine Wiedergabegeschwindigkeit, einen Wiedergabelautstärke, eine Farbe, einen Kontrast, eine Auflösung, ein für die Wiedergabe genutzter Flächenanteil des Anzeigemittels und/oder eine Wiedergabefrequenz. Für einen dieser oder mehrerer dieser Wiedergabeparameter bestimmt der Controller entsprechende Werte, sodass der Steuereinheit der wertmäßig bestimmte Wiedergabeparametersatz zugeführt werden kann.

Basierend auf dem bestimmten Wiedergabeparametersatz kann die Steuereinheit der Steuereinrichtung den bildhaften Inhalt parametrisiert auf dem Anzeigemittel wiedergeben.

Der Controller implementiert beispielsweise einen oder mehrere Algorithmen, sodass die Umwandlung des Erfassungsergebnisses in den Wiedergabeparametersatz in deterministischer Weise erfolgt. Die eingesetzten Algorithmen können in Abhängigkeit neuer Erkenntnisse angepasst werden. In dem Algorithmus ist also beispielsweise festgelegt, bei welcher Kombination von Symptomen und/oder bei welchem Ausprägungsmaß eines oder mehrerer Symptome wann und welche Anpassung des bildhaften Inhalts zu erfolgen hat.

### Steuereinheit

Die Steuereinheit gibt in Abhängigkeit von dem Wiedergabeparametersatz den parametrisierten bildhaften Inhalt auf dem Anzeigemittel zum Zwecke der Erzeugung der visuellen Stimuli aus. Mit anderen Worten: Die Steuereinheit generiert aus dem bei ihr eingehenden Wiedergabeparametersatz den parametrisierten bildhaften Inhalt.

Ferner kann die Steuereinheit die Beleuchtungseinrichtung bedienen, was bereits oben erläutert worden ist.

Nachfolgend werden weitere beispielhafte Ausführungsformen beschrieben. Die zusätzlichen Merkmale dieser weiteren Ausführungsformen können miteinander als auch mit den bereits oben beschriebenen optionalen Merkmalen zum Bilden weiterer Ausführungsbeispiele kombiniert werden, sofern sie nicht ausdrücklich als alternativ zueinander beschrieben sind.

Bei einer Ausführungsform der Steuereinrichtung umfasst die Steuereinrichtung ferner eine an den Controller gekoppelte Speichereinheit zum Speichern von bildhaften Inhalt, wobei der Controller ausgebildet ist, den gespeicherten bildhaften Inhalt in Abhängigkeit von dem Erfassungsergebnis und/oder von den Benutzereingaben zu parametrisieren und den parametrisierten bildhaften Inhalt mittels der Steuereinheit auf dem Anzeigemittel der Wiedergabeeinrichtung zum Erzeugen der visuellen Stimuli auszugeben. In der Speichereinheit sind beispielsweise bestimmte Dateien gespeichert, die Basistypen von bildhaften Inhalt beinhalten, beispielsweise eine Vielzahl von Contentelementen, die durch den Controller in Abhängigkeit von dem Erfassungsergebnis parametrisiert werden können.

Zum einen ist es also möglich, dass der Controller und die Steuereinheit das Erfassungsergebnis unabhängig vom vorgespeicherten bildhaften Inhalt selbsttätig in den parametrisierten bildhaften Inhalt wandeln, und zum anderen ist es - alternativ oder zusätzlich dazu - möglich, dass die Steuereinheit für diese Zwecke auf die optional vorgesehene Speichereinheit zugreift, um bestimmte Dateien mit bildhaften Inhalt bei der Erzeugung des parametrisierten bildhaften Inhalts einzubeziehen.

Selbstverständlich kann die Speichereinheit auch als Teil des Controllers ausgebildet sein.

Bei einer weiteren Ausführungsform der Steuereinrichtung ist die Sensoreinrichtung ausgebildet, die Sensorinformationen über das wenigstens eine Symptom der Person während der Wiedergabe des parametrisierten bildhaften Inhalts zu ermitteln, sodass Aktionen der Person -die den visuellen Stimuli ausgesetzt ist-die Bestimmung des Werts des Wiedergabeparametersatzes durch den Controller beeinflussen. Diese Ausführungsform erlaubt also eine Art Biofeedback, bei dem die Parametrisierung des bildhaften Inhalts nicht statisch erfolgt, sondern durch Einbezug aktueller Handlungen und Zustandsänderungen der Person.

Bei einer weiteren Ausführungsform der Steuereinrichtung weist die Steuereinrichtung ferner einen Datenlogger auf, der ausgebildet ist zum Aufzeichnen der personenbezogenen Daten und/oder der Umgebungsdaten, wobei die Erfassungseinrichtung beispielsweise ausgebildet ist, das Erfassungsergebnis auch in Abhängigkeit von den aufgezeichneten personenbezogenen Daten und/oder in Abhängigkeit von den aufgezeichneten Umgebungsdaten zu produzieren.

Der Datenlogger zeichnet beispielsweise die personenbezogenen Daten und/oder Umgebungsdaten während eines vorbestimmten Zeitintervalls von beispielsweise einigen Minuten, Stunden oder Tagen auf. Diese aufgezeichneten Daten bilden beispielsweise ebenfalls die Grundlage für die Bestimmung des Erfassungsergebnisses. Bevorzugt ist der Datenlogger ausgebildet, die personenbezogenen Daten verschlüsselt zu speichern, sodass Datenschutzrichtlinien berücksichtigt werden können.

Einen weiteren Aspekt bildet eine Wiedergabeeinrichtung zum Wiedergeben von bildhaften Inhalt auf einem Anzeigemittel der Wiedergabeeinrichtung, um visuelle Stimuli für eine Person zu erzeugen. Die Wiedergabeeinrichtung umfasst eine Steuereinrichtung gemäß dem ersten Aspekt. Beispielsweise ist die Wiedergabeeinrichtung für eine Anordnung in einem Patientenzimmer ausgebildet, z.B. in einem Aufwachraum für einen Komapatienten.

Einen noch weiteren Aspekt bildet ein Computerprogramm zum Betreiben einer Wiedergabeeinrichtung, aufweisend maschinenlesbaren Code, der, wenn er auf einer Steuereinrichtung der Wiedergabeeinrichtung ausgeführt wird, ausgebildet ist, die Wiedergabeeinrichtung zu veranlassen, besagtes Verfahren durchzuführen.

Die eben beschriebenen weiteren Aspekte der vorliegenden Erfindung teilen die Vorteile der Steuereinrichtung des ersten Aspektes und weisen Ausführungsformen auf, die den oben beschriebenen Ausführungsformen der Steuereinrichtung entsprechen, insbesondere, wie sie in den abhängigen Ansprüchen angegeben sind. Insoweit wird auf das Vorstehende verwiesen.

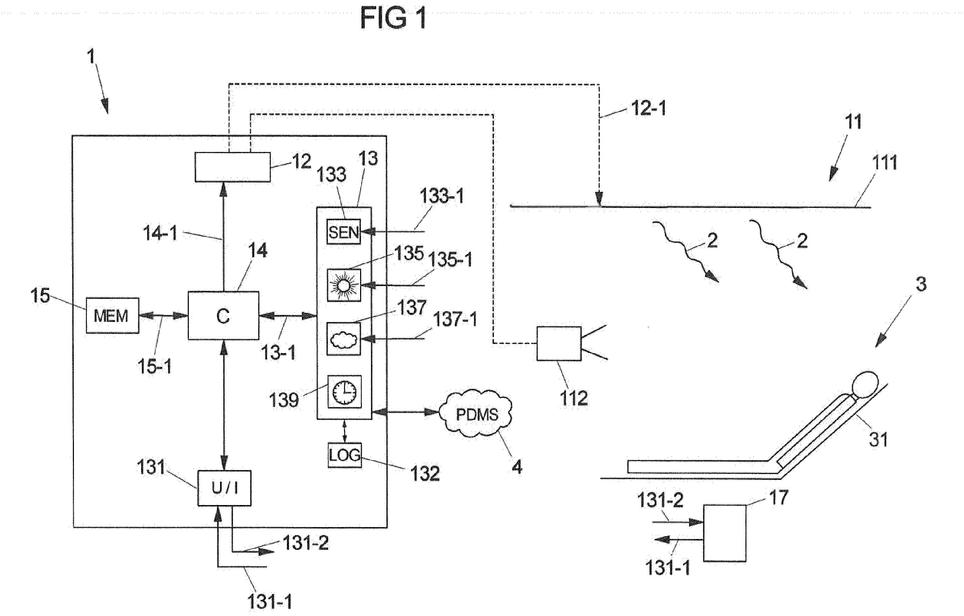

Die Fig. 1 zeigt eine schematische und exemplarische Darstellung einer Ausführungsform einer Steuereinrichtung 1. Die Steuereinrichtung 1 steuert eine Wiedergabeeinrichtung 11, die parametrisierten bildhaften Inhalt 12-1 auf einem Anzeigemittel 111 der Wiedergabeeinrichtung 11 wiedergibt, um visuelle Stimuli 2 für eine Person 3 zu erzeugen. Bei dem dargestellten Beispiel handelt es sich bei der Person 3 um einen Patienten 3, der auf einer Liege 31 liegt.

Beispielsweise soll der Patient 3 nach einem Koma einen Aufwachprozess durchlaufen. Um diesen Aufwachprozess zu unterstützen, wird der Patient visuellen Stimuli 2 ausgesetzt, deren Produktion die Steuereinrichtung 1 kontrolliert. In dem Blickfeld des Patienten 3 ist das Anzeigemittel 111 angeordnet, das in der Fig. 1 nur schematisch dargestellt ist. Beispielsweise ist das Anzeigemittel 111 an einer Zimmerdecke und/oder an einer Zimmerwand angeordnet.

Nach einer Ausführungsform weist die Steuereinrichtung 1 eine Erfassungseinrichtung 13 zum Erfassen von personenbezogenen Daten auf, die indikativ für wenigstens ein Symptom des Patienten 3 sind, und zum Erfassen von Umgebungsdaten, wobei die Erfassungseinrichtung 13 in Abhängigkeit von den personenbezogenen Daten und Umgebungsdaten ein Erfassungsergebnis 13-1 bereitstellt. Dieses Erfassungsergebnis 13-1 ist einem Controller (C) 14 zugeführt, der einen Wert eines Wiedergabeparametersatzes 14-1 in Abhängigkeit von dem Erfassungsergebnis 13-1 bestimmt. An den Controller 14 gekoppelt ist eine Steuereinheit 12, die den wertmäßig bestimmten Wiedergabeparametersatz 14-1 empfängt und die Wiedergabe des in Abhängigkeit von dem bestimmten Wiedergabeparametersatz 14-1 parametrisierten bildhaften Inhalts 12-1 auf dem Anzeigemittel 111 steuert.

Bei dem gezeigten Beispiel umfasst die Erfassungseinrichtung 13 zum Erfassen der personenbezogenen Daten zum einen eine Benutzerschnittstelle (U/I) 131, die Benutzereingaben 131-1 eines Benutzers der Steuereinrichtung 1 empfängt, wobei die Benutzereingaben 131-1 das wenigstens eine Symptom des Patienten 3 betreffen. Darüber hinaus umfasst die Erfassungseinrichtung 13 eine Sensoreinrichtung (SEN) 133, die für eine (in den Figuren nicht dargestellte) Ankopplung an den Patienten 3 ausgestaltet ist, um Sensorinformationen 133-1 über das wenigstens eine Symptom des Patienten 3 zu ermitteln.

Ferner ist ein Eingabegerät 17 vorgesehen, das zum Produzieren und Aussenden der Benutzereingaben 131-1 ausgebildet ist. Die besagten personenbezogenen Daten umfassen also zum einen die manuell eingegeben Benutzereingaben 131-1 eines Benutzers der Steuereinrichtung 1 und zum anderen die Informationen 133-1 über das wenigstens eine Symptom der Person 3, die die Erfassungseinrichtung 13 selbsttätig mittels der Sensoreinrichtung 133 ermittelt.

Der Benutzer der Steuereinrichtung 1 kann zum einen eine Person sein, die den Patienten 3 behandelt, und zum anderen aber auch der Patient 3 selbst. Beispielsweise gibt eine den Patienten 3 behandelnden Person in das Eingabegerät 17 Daten ein, die für den Zustand des Patienten 3 relevant sind. Dies erfolgt beispielsweise durch Befragung des Patienten 3 gemäß einem bestimmten Befragungsschema, das auf dem Eingabegerät 17 ausgeführt wird. Für diese Zwecke kann das Eingabegerät 17 auch drahtlos von der Steuereinrichtung 1 mittels entsprechender Befehle 131-2 gesteuert werden, um den Ablauf der Befragung des Patienten 3 zu steuern. Bei dem Eingabegerät 17 handelt es sich beispielsweise um einen Tablet-Computer oder um ein sonstiges mobiles Endgerät. Das Eingabegerät 17 leitet die Benutzereingaben 131-1 an die Benutzerschnittstelle 131 der Steuereinrichtung 1 weiter.

Die Sensoreinrichtung 133 kann eine Vielzahl von in der Fig. 1 nicht gezeigten Sensoren/Messinstrumenten aufweisen, wobei jeder der Sensoren ausgebildet ist zum Bestimmen eines Wertes einer bestimmten Körpermessgröße, und wobei der Controller 14 ausgebildet ist, eine Anzahl der Vielzahl von Sensoren auszuwählen, beispielsweise in Abhängigkeit von den empfangenen Benutzereingaben 131-1, um Werte einer Anzahl ausgewählter Körpermessgrößen zu bestimmen, und wobei die Erfassungseinrichtung 13 ausgebildet ist, das Erfassungsergebnis 13-1 in Abhängigkeit von bestimmten Werten bereitzustellen. Beispielsweise sind also eine Vielzahl von Sensoren/Messinstrumenten vorgesehen, von denen eines oder mehrere durch den Controller 14 ausgewählt werden, um weitere personenbezogenen Daten in Gestalt von Sensorinformationen 133-1 über das wenigstens eine Symptom des Patienten 3 durch die ausgewählten Sensoren/Messinstrumente zu ermitteln.

Im Ergebnis dienen die Einheiten 131 und 133 dazu, basierend auf manuellen Benutzereingaben 131-1 und basierend auf gemessenen Werten bestimmter Körpermessgrößen bestimmte Symptome und/oder Syndrome des Patienten 3 zu ermitteln. Zu derartigen Symptomen gehören z.B. die Folgenden: Agitation, Angst, Delir, Desorientierung, Halluzination, Schmerz, und/oder Sedierung.

Derartige Symptome bzw. Symptomkombinationen können bei dem Patienten 3 vorliegen und sollen mittels der visuellen Stimuli 2 beeinflusst werden. Wie die visuellen Stimuli 2 ausgeprägt sind, sprich wie der auf dem Anzeigemittel 111 wiederzugebene bildhafte Inhalt parametrisiert wird, hängt davon ab, welche Symptome bzw. Symptomkombinationen der Patient 3 zeigt und in welcher Stärke diese Symptome bzw. Symptomkombinationen vorliegen. Um also den Patienten 3 positiv durch die visuellen Stimuli 2 zu beeinflussen, ist eine exakte Identifikation und Bestimmung der Symptome durch die Erfassungseinrichtung 13 zweckmäßig. Das von der Erfassungseinrichtung 13 bereitgestellte Erfassungsergebnis 13-1 charakterisiert den Zustand des Patienten 3 hinsichtlich der bei dieser vorliegenden Symptome.

Wie in der Fig. 1 dargestellt, enthält die Erfassungseinrichtung 13 zusätzlich einen Datenlogger 132, der beispielsweise die personenbezogenen Daten 131-1 und 133-1 über einen bestimmten Zeitraum kontinuierlich aufzeichnet. Auch die aufgezeichneten personenbezogenen Daten können als Basis zur Parametrisierung des bildhaften Inhalts dienen.

Um weitere personenbezogene Daten zu ermitteln, kann die Erfassungseinrichtung 13 ferner an ein Patientendatenmanagementsystem (PDMS) 4 gekoppelt sein. Auf diesem System 4 können weitere Daten, die den Patienten 3 beschreiben, hinterlegt sein.

Für die Parametrisierung des bildhaften Inhalts 12-1 sind nicht nur die personenbezogenen Daten 131-1 und 133-1 von Relevanz, sondern auch Umgebungsdaten 135-1 und 137-1, die die unmittelbare Umgebung des Patienten 3 charakterisieren. Für diese Zwecke umfasst die Erfassungseinrichtung 13 einen Lichtsensor 135, der eine Lichtstärke und/oder eine Lichttemperatur eines Umgebungslichts 135-1, das den Patienten 3 umgibt, bestimmt. So kann beispielsweise der Kontrast in Abhängigkeit von der Lichtstärke und/oder der Lichttemperatur angepasst werden. Darüber hinaus stellt das Anzeigemittel 111 selbst eine nicht unerhebliche Lichtquelle dar, wobei der Patienten 3 in bestimmten Fällen nicht einer Lichtstärke und/oder einer Lichttemperatur ausgesetzt werden darf, die einen bestimmten maximalen Wert oder einem bestimmten minimalen Wert überschreitet bzw. unterschreitet. Aufgrund des Lichtsensors 135 können derartige Vorgaben in vorteilhafter Weise berücksichtigt werden, z.B. auch dann, wenn die Wiedergabeeinrichtung 11 nicht nur das Anzeigemittel 111 umfasst, sondern auch eine steuerbare Beleuchtungseinrichtung 112, was bereits im allgemeinen Teil der Beschreibung näher erläutert worden ist.

Außerdem umfasst die Erfassungseinrichtung 13 einen Empfänger 137 zum Empfangen von Positionsdaten und/oder Wetterdaten 137-1, die indikativ für das aktuelle und/oder zukünftige Wetter in der Umgebung des Patienten 3 sind bzw. indikativ für den Aufenthaltsort des Patienten 3. Der Empfänger 137 kann für diese Zwecke beispielsweise an das (in der Fig. 1 nicht dargestellte) Internet oder an ein (ebenfalls nicht dargestelltes) Intranet gekoppelt sein, um die betreffenden Daten zu ermitteln. Der Empfänger 137 kann aber auch entsprechende Mittel umfassen, um die Positionsdaten bzw. Wetterdaten 137-1 selbsttätig zu ermitteln, beispielsweise also einen GPS-Empfänger und/oder eine Wetterstation.

Schließlich umfasst die Erfassungseinrichtung 13 eine Zeitmesseinrichtung 139, die beispielsweise die aktuelle Uhrzeit angibt.

Basierend auf den personenbezogenen Daten und auf den Umgebungsdaten ermittelt die Erfassungseinrichtung 13 das Erfassungsergebnis 13-1. Das Erfassungsergebnis 13-1 liegt beispielsweise in Gestalt eines Datensatzes vor, der zum einen angibt, welche Symptomen bzw. welche Symptomkombination der Patient 3 aufweist, in welchem Ausprägungsmaß diese vorliegen und wie die Umgebung des Patienten 3 ausgestaltet ist.

Der Controller 14 empfängt das Erfassungsergebnis 13-1 und bestimmt darauf basierend den Wiedergabeparametersatz 14-1. Der Wiedergabeparametersatz 14-1 umfasst wenigstens einen Wiedergabeparameter, beispielsweise: einen Wiedergabeinhalt, eine Wiedergabegeschwindigkeit, eine Wiedergabelautstärke, eine Farbe, einen Kontrast, eine Auflösung, einen für die Wiedergabe genutzten Flächenanteil des Anzeigemittels und/oder eine Wiedergabefrequenz. Der Controller 14 bestimmt also, in welcher Art und Weise welcher bildhafte Inhalt auf dem Anzeigemittel 111 wiederzugeben ist.

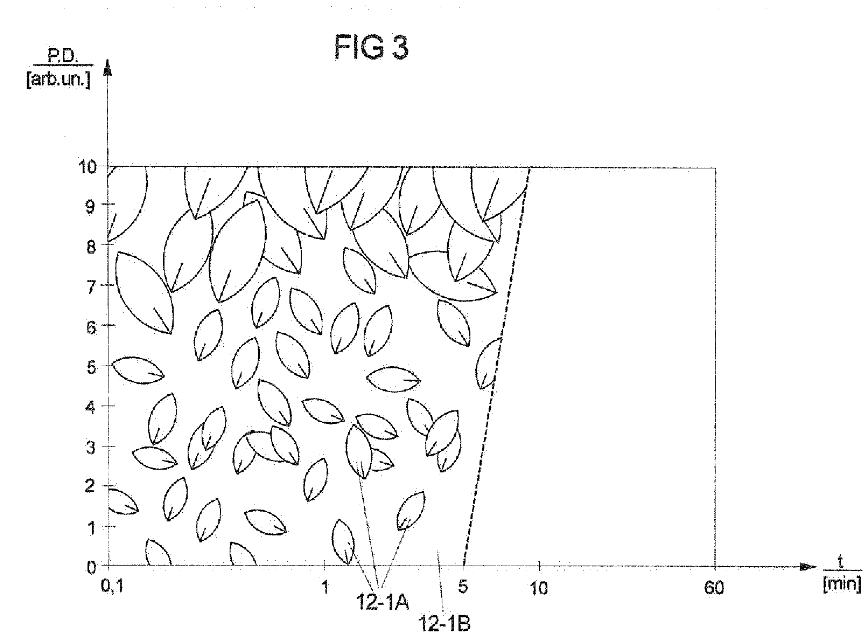

Ein Beispiel für eine Parametrisierung ist in der Fig. 3 gezeigt.

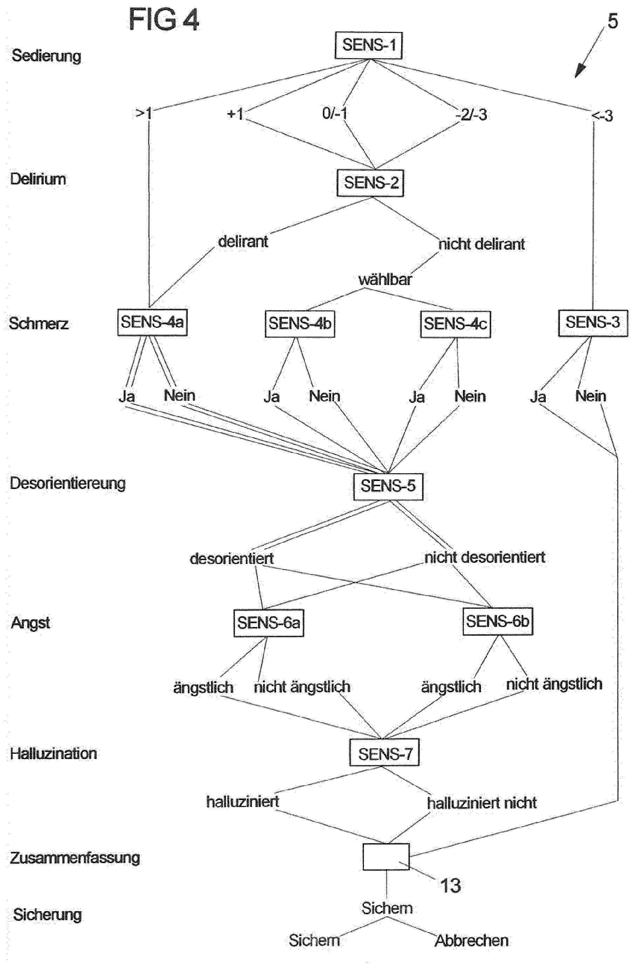

Im Wesentlichen sind dort zwei Contentelemente 12-1A und 12-1B gezeigt. Bei dem ersten Contentelement 12-1A handelt es sich um Laubblätter und bei dem zweiten Contentelement 12-1B handelt es sich um eine Hintergrundausgestaltung. Bei dem in der Fig. 4 dargestellten Diagramm ist auf der Abszissenachse die Zeit in Minuten aufgetragen und auf der Ordinatenachse personenbezogene Daten (P.D.) in einer beliebigen Einheit (arb.un.).

Beispielsweise ist auf der Ordinatenachse die Intensität eines Schmerzempfindens bei dem Patienten 3 dargestellt, wobei Null für einen geringen Schmerz steht und 10 für einen großen Schmerz. Bei dem gezeigten Beispiel nimmt der Controller 14 also eine Parametrisierung dahingehend vor, dass mit steigendem Schmerz die Größe der Blätter 12-1A zunimmt und mit zunehmender Zeit die Dichte der auf dem Hintergrund 12-1B dargestellten Blätter 12-1A. Die Farbe des Hintergrunds 12-1B verändert sich nicht in Abhängigkeit von der Schmerzintensität, sondern lediglich in Abhängigkeit von der Zeit; mit zunehmender Zeit wird der Hintergrund 12-1B dunkler.

Anhand des in der Fig. 3 gezeigten Beispiels sollte dargestellt werden, wie eine beispielhafte Parametrisierung durch den Controller 14 erfolgen kann. Wie der bildhafte Inhalt zu parametrisieren ist, bestimmt der Controller 14 durch den Wiedergabeparametersatz 14-1, der der Steuereinheit 12 zugeführt ist. Die Steuereinheit 12 gibt den parametrisierten bildhaften Inhalt 12-1 auf dem Anzeigemittel 111 aus.

Bei dem in der Fig. 1 gezeigten Beispiel umfasst die Steuereinrichtung 1 ferner eine Speichereinheit (MEM) 15, die an den Controller 14 gekoppelt ist. Auf der Speichereinheit 15 sind beispielsweise Dateien mit vorbestimmtem bildhaften Inhalt 15-1 abgelegt, auf die der Controller 14 zugreifen kann, um diese in Abhängigkeit von dem Erfassungsergebnis 13-1 zu parametrisieren. Die Speichereinheit 15 ist jedoch eine optionale Einheit der Steuereinrichtung 1.

Grundsätzlich sind die Erfassungseinrichtung 13, der Controller 14 und die Steuereinheit 12 in ihrer Gemeinsamkeit ausgebildet, basierend auf den Eingabedaten 131-1, 133-1, 135-1 und 137-1 und gegebenenfalls basierend auf Daten, die von dem Patientendatenmanagementsystem 4 empfangen werden, den parametrisierten bildhaften Inhalt 12-1 zu produzieren. Besagte Eingabedaten werden also vollautomatisch und deterministisch in den parametrisierten bildhaften Inhalt 12-1 transformiert.

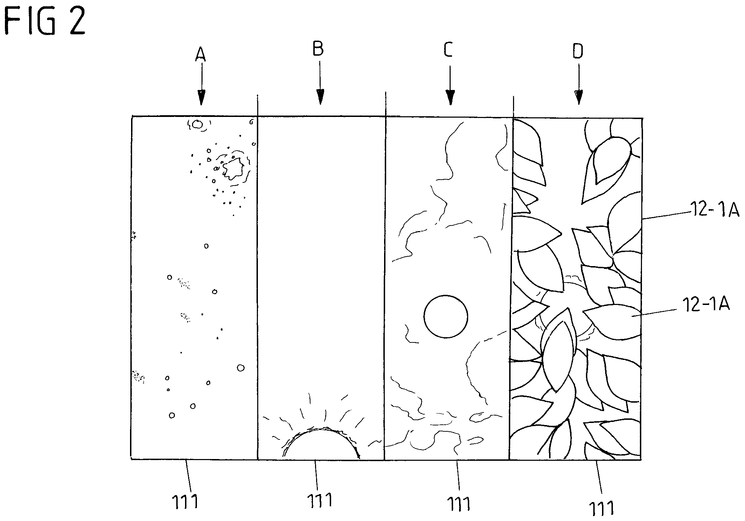

In der Fig. 2 ist schematisch und exemplarisch bildhafter Inhalt dargestellt, der von dem Anzeigemittel 111 wiedergegeben wird und in Abhängigkeit von der Uhrzeit parametrisiert worden ist. Die Uhrzeit wird durch die Zeitmesseinrichtung 139 ermittelt.

Zur Nachtzeit wird beispielsweise ein Contentelement in Gestalt eines Sternenhimmels angezeigt (Variante A). Variante B zeigt bildhaften Inhalt umfassend eine aufgehende Sonne, die beispielsweise zur Morgenzeit auf dem Anzeigemittel 111 dargestellt wird. In Abhängigkeit von den Wetterdaten 137-1 wird beispielsweise zur Tageszeit eine Sonne mit Wolkenhintergrund dargestellt (siehe Variante C) und zu einem späteren Zeitpunkt wird zusätzlich zur Sonne besagtes erstes Contentelement 12-1A in Gestalt von Laubwerk angezeigt (siehe Variante D). Die Fig. 2 zeigt jedoch nur eine Parametrisierung in Abhängigkeit von Uhrzeiten, nicht jedoch eine Parametrisierung in Abhängigkeit von personenbezogenen Daten 131-1 und 133-1, wobei eine derartige Parametrisierung in jedem Fall erfolgt. Zusätzlich zur Parametrisierung des bildhaften Inhalts in Abhängigkeit der personenbezogenen Daten 131-1 und 133-1 kann jedoch auch eine Parametrisierung in Abhängigkeit von Umgebungsdaten erfolgen, was mit Bezug auf das Beispiel gemäß der Fig. 2 anhand von Umgebungsdaten in Gestalt von Zeitdaten erläutert worden ist.

Schließlich zeigt die Fig. 4 anhand eines beispielhaften Flussdiagramms 5, wie der Controller 14 in Abhängigkeit von den Benutzereingaben 131-1 und/oder in Abhängigkeit von den Sensorinformationen 133-1 eine Reihenfolge der einzusetzenden Sensoren der Sensormesseinrichtung 133, beispielsweise zertifizierte Messinstrumente, bestimmt um weitere personenbezogene Daten in Gestalt weiterer Benutzereingaben 131-1 und/oder weiterer Sensorinformationen 133-1 zu erhalten, um also beispielsweise die bei der Person 3 vorliegenden Symptome zu ermitteln:
Bei diesem Ausführungsbeispiel umfasst die Sensoreinrichtung 133 die Sensoren SENS-1 bis SENS-7. Obwohl nachstehend jetzt von "Sensoren" die Rede ist, kann es sich bei den Sensoren z.B. um zertifizierte Messinstrumente oder andere Messgeräte handeln.

Um die bei der Person 3 vorliegenden Symptome zu ermitteln, wird zunächst ein erster Sensor SENS-1 eingesetzt, um einen Grad einer Sedierung zu ermitteln. Der Controller 14 bestimmt also, dass zunächst der erste Sensor SENS-1 entsprechende Sensorinformationen 133-1 bereitstellen muss. Der erste Sensor SENS-1 erfasst also den Wert einer Körpermessgröße, die indikativ für den Grad der Sedierung ist. Ist der Grad der Sedierung beispielsweise größer als 1, so bestimmt der Controller 14, dass in einem nächsten Schritt der Sensor SENS-4a zu verwenden ist, um einen Grad eines Schmerzes der Person 3 zu ermitteln. Liegt der Grad der Sedierung indes in dem Bereich zwischen +1 und -3, so bestimmt der Controller 14, dass in einem nächsten Schritt mittels des Sensors SENS-2 ermittelt werden soll, ob die Person delirant ist oder nicht. Ist der Grad der Sedierung kleiner als -3, so bestimmt der Controller 14, dass in einem nächsten Schritt der Sensor SENS-3 zu verwenden ist, um wiederum einen Grad eines Schmerzes der Person 3 zu ermitteln.

Der Sensor SENS-2 der Sensoreinrichtung 133 bestimmt, ob die Person 3 delirant ist oder nicht. Ist dies der Fall, so wird in einem nächsten Schritt der Sensor SENS-4a ausgewählt um wiederum den Grad eines Schmerzes der Person 3 zu ermitteln. Ist die Person nicht delirant, so wird entweder der Sensor SENS-4b oder der Sensor SENS-4c ausgewählt, um wiederum einen anderen Grad eines Schmerzes der Person 3 zu ermitteln. Die Sensoren SENS-4a, SENS-4b, SENS-4c und SENS-3 dienen also allesamt dazu, zu ermitteln, ob ein bestimmter Typ eines Schmerzes bei der Person 3 vorliegt oder nicht. In Abhängigkeit von den Ergebnissen dieser Sensoren bestimmt der Controller 14 den sodann einzusetzenden Sensor.

Beispielsweise bestimmt der Controller 14, dass in einem nächsten Schritt mittels des Sensors SENS-5 zu ermitteln ist, ob die Person 3 desorientiert ist oder nicht desorientiert. In Abhängigkeit von dem Ergebnis wird sodann mittels der Sensoren SENS-6a und SENS-6b überprüft, ob sich die Person 3 in einem Angstzustand befindet. Wiederum sind für die Ermittlung dieses Symptoms zwei voneinander verschiedene Sensoren SENS-6a und SENS-6b vorgesehen.

Schließlich wird mit dem Sensor SENS-7 überprüft, ob die Person 3 halluziniert oder nicht halluziniert.

Die gesammelten Messergebnisse sind der Erfassungseinrichtung 13 in Gestalt der Sensorinformationen 133-1 zugeführt. Zu Archivierungszwecken können die während der Messreihe aufgezeichneten Messergebnisse beispielsweise in dem Datenlogger 132 gesichert werden.

Die mittels der in der Fig. 4 dargestellten Messreihe gewonnen Sensorinformationen 133-1, die z.B. in der dargestellten Reihenfolge ermittelt worden sind, haben unmittelbaren Einfluss auf das Erfassungsergebnis 13-1 und damit auf den Wiedergabeparametersatz 14-1, der die Art und Weise der Parametrisierung des bildhaften Inhalts festlegt. Je nachdem, in welcher Reihenfolge die einzelnen Sensoren SENS-1 bis SENS-7 der Sensoreinrichtung 133 bedient werden, unterscheiden sich die gewonnen Sensorinformationen 133-1 und damit auch der bildhafte Inhalt 12-1, der zum Erzeugen der visuellen Stimuli 2 auf dem Anzeigemittel 111 wiedergegeben wird.

Die Steuereinrichtung 1 muss nicht in Gestalt eines integrierten Moduls implementiert sein; vielmehr können die Komponenten, wie beispielsweise die Steuereinheit 12, der Controller 14, der Speicher 15, die Erfassungseinheit 13 mit ihren Untereinheiten 131, 132, 133, 135, 137 und 139 auch verteilt voneinander angeordnet sein. Die einzelnen Komponenten können wahlweise drahtgebunden oder drahtlos aneinander gekoppelt sein. Insbesondere ist es beispielsweise möglich, dass die Sensoreinrichtung 133 eine Vielzahl von Messinstrumenten/Sensoren umfasst, die verteilt angeordnet sind und beispielsweise am Patienten 3 angekoppelt sind und Messwerte an die Steuereinrichtung 1 übermitteln, beispielsweise drahtlos oder drahtgebunden.

### Literaturstellen

[1] Seong-Hyun Park and Richard H Mattson. Ornamental indoor plants in hospital rooms enhanced health outcomes of patients recovering from surgery. J Altern Complement Med, 15(9):975-80, Sep 2009
[2] R S Ulrich. View through a window may influence recovery from surgery. Science, 224(4647):420-1, Apr 1984
[3] RS Ulrich, O Lunden, and Etinge JL. Effects of exposure to nature and abstract pictures on patients recovery from heart surgery. Psychophysiology, pages S1-7, 1993
[4] Quinn M. Biggs, Kimberly S. Kelly, and J. David Toney. The effects of deep diaphragmatic breathing and focused attention on dental anxiety in a private practice setting. J Dent Hyg, 77(2):105-13, 2003
[5] R. Colombo, A. Corona, F. Praga, C. Minari, C. Giannotti, A. Castelli, and F. Raimondi. A reorientation strategy for reducing delirium in the critically ill. results of an interventional study. Minerva Anestesiol, 78(9):1026-33, 9 2012
[6] S Kühn, T Gleich, R C Lorenz, U Lindenberger, and J Gallinat. Playing super mario induces structural brain plasticity: gray matter changes resulting from training with a commercial video game. Mol Psychiatry, Oct 2013
[7] J A Anguera, J Boccanfuso, J L Rintoul, O Al-Hashimi, F Faraji, J Janowich, E Kong, Y Larraburo, C Rolle, E Johnston, and A Gazzaley. Video game training enhances cognitive control in older adults. Nature, 501(7465):97-101, Sep 2013
[8] Aimee Spector, Lene Thorgrimsen, Bob Woods, Lindsay Royan, Steve Davies, Margaret Butterworth, and Martin Orrell. Efficacy of an evidence-based cognitive stimulation therapy programme for people with dementia: randomised controlled trial. Br J Psychiatry, 183:248-54, 9 2003
[9] Chia-Min M. Cheng, Ming-Jang J. Chiu, Jyh-Horng H. Wang, Hwa-Chang C. Liu, Yea-Ing Lotus I. Shyu, Guan-Hua H. Huang, and Cheryl Chia-Hui Chen. Cognitive stimulation during hospitalization improves global cognition of older taiwanese undergoing elective total knee and hip replacement surgery. J Adv Nurs, 68(6):1322-9, 6 2012

## Patentansprüche

1. Steuereinrichtung (1) zum Steuern einer Wiedergabeeinrichtung (11), die ausgebildet ist zum Wiedergeben von bildhaften Inhalt auf einem Anzeigemittel (111) der Wiedergabeeinrichtung (11), um visuelle Stimuli (2) für eine Person (3) zu erzeugen, wobei die Steuereinrichtung (1) umfasst:
- eine Erfassungseinrichtung (13), die ausgebildet ist zum Erfassen von personenbezogenen Daten (131-1; 133-1), die indikativ für wenigstens ein Symptom der Person (3) sind, und zum Bereitstellen eines Erfassungsergebnisses (13-1) in Abhängigkeit von den personenbezogenen Daten, wobei die Erfassungseinrichtung (13) zum Erfassen der personenbezogenen Daten (131-1; 133-1) aufweist:
- eine Sensoreinrichtung (133), die ausgestaltet ist für eine Ankopplung an die Person (3), um personenbezogenen Daten in Gestalt von Sensorinformationen (133-1) über das wenigstens eine Symptom der Person (3) zu ermitteln, wobei die Sensoreinrichtung (133) eine Vielzahl von Sensoren (SENS) aufweist, wobei die Sensorinformationen (133-1) über das wenigstens eine Symptom Werte von Körpermessgrößen umfassen, wobei jeder der Sensoren (SENS) ausgebildet ist zum Bestimmen eines Werts einer bestimmten Körpermessgröße;
- einen Controller (14), der ausgebildet ist zum Bestimmen wenigstens eines Werts eines Wiedergabeparametersatzes (14-1) in Abhängigkeit von dem Erfassungsergebnis (13-1); und
- eine Steuereinheit (12), die ausgebildet ist zum Steuern der Wiedergabe eines in Abhängigkeit von dem wertmäßig bestimmten Wiedergabeparametersatz (14-1) parametrisierten bildhaften Inhalts (12-1);
**dadurch gekennzeichnet, dass**
- der Controller (14) weiter ausgebildet ist, eine Anzahl der Vielzahl von Sensoren (SENS) auszuwählen in Abhängigkeit von zuvor durch die Sensoreinrichtung (133) ermittelten Sensorinformationen (133-1), um Werte einer Anzahl ausgewählter Körpermessgrößen zu bestimmen; und
- die Erfassungseinrichtung (13) ausgebildet ist, das Erfassungsergebnis (13-1) in Abhängigkeit von den bestimmten Werten der Anzahl ausgewählter Körpermessgrößen bereitzustellen.

2. Steuereinrichtung (1) nach Anspruch 1, wobei die Erfassungseinrichtung (13) zum Erfassen der personenbezogenen Daten (131-1; 133-1) aufweist:
- eine Benutzerschnittstelle (131), die ausgebildet ist zum Empfangen von personenbezogenen Daten in Gestalt von Benutzereingaben (131-1) eines Benutzers der Steuereinrichtung (1), wobei die Benutzereingaben (131-1) das wenigstens eine Symptom der Person (3) betreffen.

3. Steuereinrichtung (1) nach Anspruch 2, wobei der Controller (14) ausgebildet ist, eine Anzahl der Vielzahl von Sensoren (SENS) auszuwählen in Abhängigkeit von den empfangenen Benutzereingaben (131-1).

4. Steuereinrichtung (1) nach einem der vorstehenden Ansprüche, wobei der Controller (14) ausgebildet ist, eine Reihenfolge der einzusetzenden Sensoren der Sensoreinrichtung (133) in Abhängigkeit von jeweils zuvor durch die Sensoreinrichtung (133) ermittelten Sensorinformationen (133-1) und/oder - insofern die Erfassungseinrichtung (13) eine Benutzerschnittstelle (131) gemäß Anspruch 2 umfasst - von den Benutzereingaben (131-1) zu bestimmen.

5. Steuereinrichtung (1) nach einem der vorstehenden Ansprüche, wobei die Erfassungseinrichtung (13) ferner ausgebildet ist zum Erfassen von Umgebungsdaten (135-1, 137-1) und zum Bereitstellen des Erfassungsergebnisses (13-1) in Abhängigkeit von den Umgebungsdaten, wobei die Erfassungseinrichtung (13) zum Erfassen der Umgebungsdaten (135-1, 137-1) aufweist:
- einen Lichtsensor (135), der ausgebildet ist zum Bestimmen einer Lichtstärke und/oder einer Lichttemperatur eines Umgebungslichts (135-1), das die Person (3) umgibt; und/oder
- einen Empfänger (137), der ausgebildet ist zum Empfangen von Wetterdaten und/oder Positionsdaten (137-1), die indikativ für das aktuelle und/oder zukünftige Wetter in der Umgebung der Person (3) sind bzw. indikativ für den Aufenthaltsort der Person (3) sind.

6. Steuereinrichtung (1) nach einem der vorstehenden Ansprüche, wobei die Steuereinrichtung (1) ferner eine an den Controller (14) gekoppelte Speichereinheit (15) zum Speichern von bildhaftem Inhalt aufweist, wobei der Controller (14) ausgebildet ist, den gespeicherten bildhaften Inhalt (15-1) in Abhängigkeit von dem Erfassungsergebnis (13-1) und/oder - insofern die Erfassungseinrichtung (13) eine Benutzerschnittstelle (131) gemäß Anspruch 2 umfasst - in Abhängigkeit von den Benutzereingaben (131-1) zu parametrisieren.

7. Steuereinrichtung (1) nach einem der vorstehenden Ansprüche, wobei die Sensoreinrichtung (133) ausgebildet ist, die Sensorinformationen (133-1) über das wenigstens eine Symptom der Person (3) während der Wiedergabe des parametrisierten bildhaften Inhalts (12-1) zu ermitteln, sodass das aktuelle wenigstens eine Symptom der Person (3) die Bestimmung des Werts des Wiedergabeparametersatzes (14-1) beeinflusst und damit die Parametrisierung des aktuell wiedergegebenen bildhaften Inhalts (12-1).

8. Steuereinrichtung (1) nach einem der vorstehenden Ansprüche 2 bis 7, wobei die Steuereinrichtung (1) ferner ein Eingabegerät (17) aufweist, das zum Produzieren und Aussenden der Benutzereingaben (131-1) an die Steuereinrichtung (1) ausgebildet ist.

9. Steuereinrichtung (1) nach Anspruch 8, wobei der Controller (14) ausgebildet ist, eine Ausführung eines Programms auf dem Eingabegerät (17) zu steuern in Abhängigkeit von den Benutzereingaben (131-1) und/oder in Abhängigkeit von dem Erfassungsergebnis (13-1).

10. Steuereinrichtung (1) nach einem der vorstehenden Ansprüche, wobei die Steuereinrichtung (1) ferner einen Datenlogger (132) aufweist, der ausgebildet ist zum Aufzeichnen der personenbezogenen Daten (131-1, 133-1) und/oder - insofern die Erfassungsrichtung gemäß Anspruch 5 zum Erfassen von Umgebungsdaten (135-1, 137-1) ausgebildet ist - der Umgebungsdaten (135-1, 137-1), wobei die Erfassungseinrichtung (13) ausgebildet ist zum Produzieren des Erfassungsergebnisses (13-1) zusätzlich in Abhängigkeit von den aufgezeichneten personenbezogenen Daten und/oder von den aufgezeichneten Umgebungsdaten.

11. Steuereinrichtung (1) nach einem der vorstehenden Ansprüche, wobei der bildhafte Inhalt parametrisch generierte Echtzeit-Bewegtbilder umfasst.

12. Steuereinrichtung (1) nach einem der vorstehenden Ansprüche, wobei der Controller (14) und die Steuereinheit (12) in ihrer Gemeinsamkeit ausgebildet sind zum Transformieren des Erfassungsergebnisses (13-1) und - insofern die Erfassungseinrichtung (13) eine Benutzerschnittstelle (131) gemäß Anspruch 2 umfasst - optional der Benutzereingaben (131-1) in den wiederzugegebenen bildhaften Inhalt (12-1).

13. Steuereinrichtung (1) nach einem der vorstehenden Ansprüche, wobei die Wiedergabeeinrichtung (11) eine Beleuchtungseinrichtung (112) zum Beleuchten der Umgebung der Person (3) aufweist, wobei der Controller (14) und die Steuereinheit (12) ausgebildet sind, die Beleuchtungseinrichtung in Abhängigkeit von dem Erfassungsergebnis (13-1) und/oder - insofern die Erfassungseinrichtung (13) eine Benutzerschnittstelle (131) gemäß Anspruch 2 umfasst - von den Benutzereingaben (131-1) zu steuern.

14. Wiedergabeeinrichtung (1, 11) zum Wiedergeben von parametrisierten bildhaften Inhalt (12-1) auf einem Anzeigemittel (111) der Wiedergabeeinrichtung (11), um visuelle Stimuli (2) für eine Person (3) zu erzeugen, umfassend eine Steuereinrichtung (1) nach einem der vorstehenden Ansprüche.

15. Computerprogramm zum Betreiben einer Wiedergabeeinrichtung, aufweisend maschinenlesbaren Code, der, wenn er auf einer Steuereinrichtung der Wiedergabeeinrichtung ausgeführt wird, ausgebildet ist, die Steuereinrichtung zu veranlassen, ein Verfahren zum Steuern der Wiedergabeeinrichtung (11) durchzuführen, wobei die Wiedergabeeinrichtung ausgebildet ist zum Wiedergeben von bildhaften Inhalt auf einem Anzeigemittel (111) der Wiedergabeeinrichtung (11), um visuelle Stimuli (2) für eine Person (3) zu erzeugen, und wobei das Verfahren (1) umfasst:
- Erfassen von personenbezogenen Daten (131-1; 133-1), die indikativ für wenigstens ein Symptom der Person (3) sind, und Bereitstellen eines Erfassungsergebnisses (13-1) in Abhängigkeit von den personenbezogenen Daten, wobei das Erfassen der personenbezogenen Daten über eine Sensoreinrichtung (133) erfolgt, die ausgestaltet ist für eine Ankopplung an die Person (3), um personenbezogenen Daten in Gestalt von Sensorinformationen (133-1) über das wenigstens eine Symptom der Person (3) zu ermitteln, wobei die Sensoreinrichtung (133) eine Vielzahl von Sensoren (SENS) aufweist, wobei die Sensorinformationen (133-1) über das wenigstens eine Symptom Werte von Körpermessgrößen umfassen, wobei jeder der Sensoren (SENS) ausgebildet ist zum Bestimmen eines Werts einer bestimmten Körpermessgröße;
- Bestimmen wenigstens eines Werts eines Wiedergabeparametersatzes (14-1) in Abhängigkeit von dem Erfassungsergebnis (13-1), wobei das Bestimmen ein Auswählen einer Anzahl der Vielzahl von Sensoren (SENS) in Abhängigkeit von zuvor durch die Sensoreinrichtung (133) ermittelten Sensorinformationen (133-1) umfasst, um Werte einer Anzahl ausgewählter Körpermessgrößen zu bestimmen;
- Bereitstellen des Erfassungsergebnisses (13-1) in Abhängigkeit von den bestimmten Werten der Anzahl ausgewählter Körpermessgrößen; und
- Steuern der Wiedergabe eines in Abhängigkeit von dem wertmäßig bestimmten Wiedergabeparametersatz (141) parametrisierten bildhaften Inhalts (12-1).

## Claims

1. Control device (1) for controlling a playback device (11), which is configured to play back pictorial content on a display means (111) of the playback device (11) to generate visual stimuli (2) for a person (3), wherein the control device (1) comprises:
- an acquisition device (13), which is configured to acquire person-related data (131-1; 133-1), which are indicative of at least one symptom of the person (3), and to provide an acquisition result (13-1) depending on the person-related data, wherein the acquisition device (13) for acquiring the person-related data (131-1; 133-1) has:
- a sensor device (133), which is embodied for a coupling to the person (3), to ascertain person-related data in the form of items of sensor information (133-1) about the at least one symptom of the person (3), wherein the sensor device (133) has a plurality of sensors (SENS), wherein the items of sensor information (133-1) about the at least one symptom comprise values of physical measured variables, wherein each of the sensors (SENS) is configured to determine a value of a specific physical measured variable;
- a controller (14), which is configured to determine at least one value of a playback parameter set (14-1) depending on the acquisition result (13-1); and
- a control unit (12), which is configured to control the playback of a pictorial content (12-1) that is parametrized depending on the playback parameter set (14-1) that is determined with respect to value;
**characterized in that**
- the controller (14) is furthermore configured to select a number of the plurality of sensors (SENS) depending on items of sensor information (133-1) which have been previously determined by the sensor device (133), to determine values of a number of selected physical measured variables; and
- the acquisition device (13) is configured to provide the acquisition result (13-1) depending on the determined values of the number of selected physical measured variable.

2. Control device (1) according to Claim 1, wherein the acquisition device (13) for acquiring the person-related data (131-1; 133-1) has:
- a user interface (131), which is configured to receive person-related data in the form of user inputs (131-1) of a user of the control device (1), wherein the user inputs (131-1) relate to the at least one symptom (3) of the person.

3. Control device (1) according to Claim 2, wherein the controller (14) is configured to select a number of the plurality of sensors (SENS) depending on the received user inputs (131-1).

4. Control device (1) according to any one of the preceding claims, wherein the controller (14) is configured to determine a sequence of the sensors of the sensor device (133) to be used depending on items of sensor information (133-1) which have respectively been determined previously by the sensor device (133) and/or - insofar as the acquisition device (13) comprises a user interface (131) according to Claim 2-on the user inputs (131-1).

5. Control device (1) according to any one of the preceding claims, wherein the acquisition device (13) is furthermore configured to acquire environmental data (135-1, 137-1) and to provide the acquisition result (13-1) depending on the environmental data, wherein the acquisition device (13) has, for acquiring the environmental data (135-1, 137-1):
- a light sensor (135), which is configured to determine a luminosity and/or a light temperature of an ambient light (135-1), which surrounds the person (3); and/or
- a receiver (137), which is configured to receive weather data and/or position data (137-1), which are indicative for the present and/or future weather in the environment of the person (3) and/or are indicative of the location of the person (3).

6. Control device (1) according to any one of the preceding claims, wherein the control device (1) furthermore has a storage unit (15) coupled to the controller (14) for storing pictorial content, wherein the controller (14) is configured to parameterize the stored pictorial content (15-1) depending on the acquisition result (13-1) and/or depending on -insofar as the acquisition device (13) comprises a user interface (131) according to Claim 2- the user inputs (131-1).

7. Control device (1) according to any one of the preceding claims, wherein the sensor device (133) is configured to ascertain the items of sensor information (133-1) about the at least one symptom of the person (3) during the playback of the parameterized pictorial content (12-1), so that the present at least one symptom of the person (3) influences the determination of the value of the playback parameter set (14-1) and therefore the parameterization of the presently played back pictorial content (12-1).

8. Control device (1) according to any one of the preceding Claims 2 to 7, wherein the control device (1) furthermore has an input device (17), which is configured for producing and transmitting the user inputs (131-1) to the control device (1).

9. Control device (1) according to Claim 8, wherein the controller (14) is configured to control an execution of a program on the input device (17) depending on the user inputs (131-1) and/or depending on the acquisition result (13-1).

10. Control device (1) according to any one of the preceding claims, wherein the control device (1) furthermore has a data logger (132), which is configured to record the person-related data (131-1, 133-1) and/or - insofar as the acquisition device according to Claim 5 is configured to acquire environmental data (135-1,137-1) - the environmental data (135-1, 137-1), wherein the acquisition device (13) is configured to produce the acquisition result (13-1) in addition depending on the recorded person-related data and/or the recorded environmental data.

11. Control device (1) according to any one of the preceding claims, wherein the pictorial content comprises parametrically generated real-time moving images.

12. Control device (1) according to any one of the preceding claims, wherein the controller (14) and the control unit (12) are configured in their commonality to transform the acquisition result (13-1) and - insofar as the acquisition device (13) comprises a user interface (131) according to Claim 2 - optionally the user inputs (131-1) into the pictorial content (12-1) to be played back.

13. Control device (1) according to any one of the preceding claims, wherein the playback device (11) has a lighting device (112) for illuminating the environment of the person (3), wherein the controller (14) and the control unit (12) are configured to control the lighting device depending on the acquisition result (13-1) and/or - insofar as the acquisition device (13) comprises a user interface (131) according to Claim 2 - on the user inputs (131-1).

14. Playback device (1, 11) for playing back parameterized pictorial content (12-1) on a display means (111) of the playback device (11), to generate visual stimuli (2) for a person (3), comprising a control device (1) according to any one of the preceding claims.

15. Computer program for operating a playback device, having machine-readable code which, when it is executed on a control device of the playback device, is configured to cause the control device to carry out a method for controlling the playback device (11), wherein the playback device is configured to play back pictorial content on a display means (111) of the playback device (11) to generate visual stimuli (2) for a person (3), and wherein the method (1) comprises:
- acquiring person-related data (131-1; 133-1), which are indicative of at least one symptom of the person (3), and providing an acquisition result (13-1) depending on the person-related data, wherein the acquisition of the person-related data is performed via a sensor device (133), which is configured for coupling to the person (3) to ascertain person-related data in the form of items of sensor information (133-1) about the at least one symptom of the person (3), wherein the sensor device (133) has a plurality of sensors (SENS), wherein the items of sensor information (133-1) about the at least one symptom comprise values of physical measured variables, wherein each of the sensors (SENS) is configured to determine a value of a specific physical measured variable;
- determining at least one value of a playback parameter set (14-1) depending on the acquisition result (13-1), wherein the determination comprises a selection of a number of the plurality of sensors (SENS) depending on items of sensor information (133-1) which have been previously determined by the sensor device (133), to determine values of a number of selected physical measured variables;
- providing the acquisition result (13-1) depending on the determined values of the number of selected physical measured variables; and
- controlling the playback of pictorial content (12-1), which is parameterized depending on the playback parameter set (14-1) that is determined with respect to value.

## Revendications

1. Dispositif de commande (1) pour commander un dispositif de reproduction (11), qui est configuré pour reproduire un contenu imagé sur un moyen d'affichage (111) du dispositif de reproduction (11), afin de générer des stimuli visuels (2) pour une personne (3), dans lequel le dispositif de commande (1) comprend:
- un dispositif de détection (13), qui est configuré pour détecter des données liées à la personne (131-1; 133-1), qui sont indicatives d'au moins un symptôme de la personne (3), et pour fournir un résultat de détection (13-1) en fonction des données liées à la personne, dans lequel le dispositif de détection (13) pour détecter les données liées à la personne (131-1; 133-1) présente:
- un dispositif de capteurs (133), qui est configuré pour un couplage à la personne (3), afin de déterminer des données liées à la personne sous la forme d'informations de capteur (133-1) au sujet dudit au moins un symptôme de la personne (3), dans lequel le dispositif de capteurs (133) présente une multiplicité de capteurs (SENS), dans lequel les informations de capteur (133-1) au sujet dudit au moins un symptôme comprennent des valeurs de grandeurs de mesure corporelles, dans lequel chacun des capteurs (SENS) est configuré pour déterminer une valeur d'une grandeur de mesure corporelle déterminée;
- un contrôleur (14), qui est configuré pour déterminer au moins une valeur d'un jeu de paramètres de reproduction (14-1) en fonction du résultat de détection (13-1); et
- une unité de commande (12), qui est configurée pour commander la reproduction d'un contenu imagé (12-1) paramétré en fonction du jeu de paramètres de reproduction (14-1) déterminé selon la valeur;
**caractérisé en ce que**
- le contrôleur (14) est configuré en outre pour sélectionner un nombre de la multiplicité de capteurs (SENS) en fonction d'informations de capteur (133-1) déterminées auparavant par le dispositif de capteurs (133), afin de déterminer des valeurs d'un nombre de grandeurs de mesure corporelles sélectionnées; et
- le dispositif de détection (13) est configuré pour fournir le résultat de détection (13-1) en fonction des valeurs déterminées du nombre de grandeurs de mesure corporelles sélectionnées.

2. Dispositif de commande (1) selon la revendication 1, dans lequel le dispositif de détection (13) pour détecter les données liées à la personne (131-1; 133-1) présente:
- une interface d'utilisateur (131), qui est conçue pour recevoir des données liées à la personne sous la forme d'entrées d'utilisateur (131-1) d'un utilisateur du dispositif de commande (1), dans lequel les entrées d'utilisateur (131-1) concernent ledit au moins un symptôme de la personne (3).

3. Dispositif de commande (1) selon la revendication 2, dans lequel le contrôleur (14) est conçu pour sélectionner un nombre de la multiplicité de capteurs (SENS) en fonction des entrées d'utilisateur reçues (131-1).

4. Dispositif de commande (1) selon l'une quelconque des revendications précédentes, dans lequel le contrôleur (14) est conçu pour déterminer une succession des capteurs du dispositif de capteurs (133) à utiliser en fonction d'informations de capteur (133-1) respectivement déterminées auparavant par le dispositif de capteurs (133) et/ou - dans la mesure où le dispositif de détection (13) comprend une interface d'utilisateur (131) selon la revendication 2 - en fonction des entrées d'utilisateur (131-1).

5. Dispositif de commande (1) selon l'une quelconque des revendications précédentes, dans lequel le dispositif de détection (13) est en outre configuré pour détecter des données ambiantes (135-1, 137-1) et pour fournir le résultat de détection (13-1) en fonction des données ambiantes, dans lequel le dispositif de détection (13) pour détecter des données ambiantes (135-1, 137-1) présente:
- un capteur de lumière (135), qui est conçu pour déterminer une intensité lumineuse et/ou une température de lumière d'une lumière ambiante (135-1), qui entoure la personne (3); et/ou
- un récepteur (137), qui est conçu pour recevoir des données météorologiques et/ou des donnés de position (137-1), qui sont indicatives du temps actuel et/ou futur dans l'environnement de la personne (3) ou qui sont indicatives du lieu de séjour de la personne (3).

6. Dispositif de commande (1) selon l'une quelconque des revendications précédentes, dans lequel le dispositif de commande (1) présente en outre une unité de mémoire (15) couplée au contrôleur (14) pour mémoriser un contenu imagé, dans lequel le contrôleur (14) est conçu pour paramétrer le contenu imagé mémorisé (15-1) en fonction du résultat de détection (13-1) et/ou - dans la mesure où le dispositif de détection (13) comprend une interface d'utilisateur (131) selon la revendication 2 - en fonction des entrées d'utilisateur (131-1).

7. Dispositif de commande (1) selon l'une quelconque des revendications précédentes, dans lequel le dispositif de capteurs (133) est conçu pour déterminer les informations de capteur (133-1) au sujet dudit au moins un symptôme de la personne (3) pendant la reproduction du contenu imagé paramétré (12-1), de telle manière que ledit au moins un symptôme actuel de la personne (3) influence la détermination de la valeur du jeu de paramètres de reproduction (14-1) et dès lors le paramétrage du contenu imagé reproduit actuel (12-1).

8. Dispositif de commande (1) selon l'une quelconque des revendications précédentes 2 à 7, dans lequel le dispositif de commande (1) présente en outre un appareil d'entrée (17), qui est conçu pour produire et envoyer les entrées d'utilisateur (131-1) au dispositif de commande (1).

9. Dispositif de commande (1) selon la revendication 8, dans lequel le contrôleur (14) est configuré pour commander une exécution d'un programme sur l'appareil d'entrée (17) en fonction des entrées d'utilisateur (131-1) et/ou en fonction du résultat de détection (13-1).

10. Dispositif de commande (1) selon l'une quelconque des revendications précédentes, dans lequel le dispositif de commande (1) présente en outre un collecteur de données (132), qui est conçu pour enregistrer les données liées à la personne (131-1, 133-1) et/ou - dans la mesure où le dispositif de détection est configuré selon la revendication 5 pour détecter des données ambiantes (135-1, 137-1) - les données ambiantes (135-1, 137-1), dans lequel le dispositif de détection (13) est configuré pour produire le résultat de détection (13-1) en outre en fonction des données liées à la personne enregistrées et/ou des données ambiantes enregistrées.

11. Dispositif de commande (1) selon l'une quelconque des revendications précédentes, dans lequel le contenu imagé comprend des images mobiles en temps réel générées de façon paramétrique.

12. Dispositif de commande (1) selon l'une quelconque des revendications précédentes, dans lequel le contrôleur (14) et l'unité de commande (12) sont dans leur globalité conçus pour transformer le résultat de détection (13-1) et en option - dans la mesure où le dispositif de détection (13) comprend une interface d'utilisateur (131) selon la revendication 2 - des entrées d'utilisateur (131-1) en ledit contenu imagé à reproduire (12-1).

13. Dispositif de commande (1) selon l'une quelconque des revendications précédentes, dans lequel le dispositif de reproduction (11) présente un dispositif d'éclairage (112) pour éclairer l'environnement de la personne (3), dans lequel le contrôleur (14) et l'unité de commande (12) sont conçus pour commander le dispositif d'éclairage en fonction du résultat de détection (13-1) et/ou - dans la mesure où le dispositif de détection (13) comprend une interface d'utilisateur (131) selon la revendication 2 - des entrées d'utilisateur (131-1).

14. Dispositif de reproduction (1, 11) pour la reproduction d'un contenu imagé paramétré (12-1) sur un moyen d'affichage (111) du dispositif de reproduction (11), afin de générer des stimuli visuels (2) pour une personne (3), comprenant un dispositif de commande (1) selon l'une quelconque des revendications précédentes.

15. Programme informatique pour faire fonctionner un dispositif de reproduction, présentant un code lisible à la machine qui, lorsqu'il est exécuté sur un dispositif de commande du dispositif de reproduction, est configuré pour amener le dispositif de commande à exécuter un procédé pour commander le dispositif de reproduction (11), dans lequel le dispositif de reproduction est conçu pour reproduire un contenu imagé sur un moyen d'affichage (111) du dispositif de reproduction (11), afin de générer des stimuli visuels (2) pour une personne (3), et dans lequel le procédé (1) comprend les étapes suivantes:
- détecter des données liées à la personne (131-1; 133-1), qui sont indicatives d'au moins un symptôme de la personne (3), et fournir un résultat de détection (13-1) en fonction des données liées à la personne, dans lequel la détection des données liées à la personne est effectuée par un dispositif de capteurs (133), qui est configuré pour un couplage à la personne (3) afin de déterminer des données liées à la personne sous la forme d'informations de capteur (133-1) au sujet dudit au moins un symptôme de la personne (3), dans lequel le dispositif de capteurs (133) présente une multiplicité de capteurs (SENS), dans lequel les informations de capteur (133-1) au sujet dudit au moins un symptôme comprennent des valeurs de grandeurs de mesure corporelles, dans lequel chacun de capteurs (SENS) est conçu pour déterminer une valeur d'une grandeur de mesure corporelle déterminée;
- déterminer au moins une valeur d'un jeu de paramètres de reproduction (14-1) en fonction du résultat de détection (13-1), dans lequel la détermination comprend une sélection d'un nombre de la multiplicité de capteurs (SENS) en fonction d'informations de capteur (133-1) déterminées auparavant par le dispositif de capteurs (133), afin de déterminer des valeurs d'un nombre de grandeurs de mesure corporelles sélectionnées;
- fournir le résultat de détection (13-1) en fonction des valeurs déterminées du nombre de grandeurs de mesure corporelles sélectionnées; et
- commander la reproduction d'un contenu imagé paramétré (12-1) en fonction du jeu de paramètres de reproduction (14-1) déterminé selon la valeur.
